# EUROPEAN PATENT APPLICATION

(11) **EP 4 530 297 A1**
(43) Date of publication of application: **02.04.2025**
(21) Application number: 23795213.0
(22) Date of filing: 21.04.2023
(51) Int. Cl.: C07K 16/28, A61K 39/395, A61P 9/10, A61P 3/06

(54) **ANTI-ASGR1 MONOCLONAL ANTIBODY AND USE THEREOF**

(30) Priority: 25.04.2022 CN 202210440150
(71) Applicant: Wuhan University, Wuhan, Hubei 430072 (CN)
(72) Inventor: SONG, Baoliang, Wuhan, Hubei 430072 (CN); WANG, Juqiong, Wuhan, Hubei 430072 (CN)
(74) Representative: Murgitroyd & Company
(86) International application number: PCT/CN2023/089737
(87) International publication number: WO 2023/207780

(57) **Abstract**

Disclosed is an anti-ASGR1 monoclonal antibody and a use thereof. Further disclosed is use of a combination of an ASGR1 inhibitor including the anti-ASGR1 monoclonal antibody and a second lipid-lowering agent in reducing the level of total cholesterol and/or triglyceride in the blood and/or liver and/or in treating and/or preventing cardiovascular diseases. The present invention confirms that the combination of the ASGR1 inhibitor and the second lipid-lowering agent has the effect of synergistically reducing the total cholesterol and triglyceride in the serum and the liver.

## Description

### TECHNICAL FIELD

The present disclosure relates to an anti-ASGR1 monoclonal antibody. The present disclosure also relates to a combinational use of an ASGR1 inhibitor (e.g., the anti-ASGR1 monoclonal antibody) and another lipid-lowering agent.

### BACKGROUND

Statins, commonly used to reduce low-density lipoprotein cholesterol (LDL-c), are the first-line drugs for reducing cholesterol. Statins currently approved for market by the FDA include lovastatin, Simvastatin, pravastatin, fluvastatin, atorvastatin, rosuvastatin, and pitavastatin, with atorvastatin and rosuvastatin being the most widely used. However, related studies have found that long-term use of statins will cause statin resistance in patients, resulting in liver toxicity. A portion of patients who are statin intolerant will have side effects such as myasthenia, rhabdomyolysis, and diabetes, etc. Medium-intensity statins cannot meet the clinical needs of intensive lipid-lowering; and after doubling the dosage of high-dose statins, the therapeutic effect is increased by only 6% while the adverse reaction is doubled, so the clinical application has great limitations.

Ezetimibe (EZ) is the first cholesterol absorption inhibitor with a primary function being to inhibit the NPC1L1-mediated absorption of cholesterol from diet and bile by the brush border of the small intestine, without affecting the absorption of other fat-soluble nutrients, and is mainly used in hypercholesterolemia. After EZ treatment, lipid accumulation in the liver is reduced, cholesterol, triglyceride, and LDL-c in the blood are reduced, and high-density lipoprotein cholesterol (HDL-c) is increased, having no significant effect on fat-soluble vitamins such as vitamin A, vitamin D, and vitamin E. However, when Ezetimibe is used alone, the plasma concentration of LDL-c is reduced to a lesser extent (about 20%), so EZ is used clinically in combination with statins. In four multicenter, double-blind, placebo-controlled 12-week studies, 1187 patients with primary hypercholesterolemia were treated with 10 mg of Ezetimibe daily alone or in combination with atorvastatin, Simvastatin, pravastatin, or lovastatin. The degree of LDL-c reduction in patients receiving combination therapy is independent of the type and dose of statins. The combinational use of Ezetimibe and minimum-dose statins has superior effect in LDL-c reduction compared to the use of high-dose statins alone. Ezetimibe/Simvastatin tablet (VYTORIN^{®}) is currently the only lipid-lowering compound preparation available in China. The tablet contains two lipid-lowering agents, Simvastatin and Ezetimibe, which are widely used in clinical applications, and is suitable for patients with hypercholesterolemia. Etimibe tablet employs a dual mechanism of action, inhibiting the absorption of cholesterol in the small intestine while also inhibiting the synthesis of cholesterol in the liver, which can effectively reduce LDL-c levels by 50% or more.

Although the combination therapy of Ezetimibe with statin lipid-lowering agents reduces the dose of statins to some extent, thereby reducing their toxic and side effects, such combination therapy still has common adverse effects, for example, abnormalities in liver function tests, such as elevated alanine transaminase (ALT) and/or aspartate transaminase (AST) levels; elevated creatine kinase (CK) levels in the blood, and uncommon elevated bilirubin levels in the blood; elevated uric acid levels in the blood; elevated γ-glutamyl transpeptidase levels; elevated international normalized ratio; presence of proteinuria; and weight loss, etc.

WO 2017058944 A1, WO 2022006327 A1, and US 20210163601 A1 disclose methods for treating and preventing cardiovascular diseases by administering ASGR-1 antigen-binding proteins, in which anti-ASGR1 antibodies can reduce the levels of low-density lipoprotein cholesterol (LDL-c) and/or non-high-density lipoprotein cholesterol (Non-HDL-c). However, there are no ASGR1 antibodies currently approved for market for the treatment of isolated hypercholesterolemia or other cardiovascular diseases.

### SUMMARY

In a first aspect, the present disclosure provides a monoclonal antibody or an antigen-binding fragment thereof that binds to ASGR1, wherein the monoclonal antibody inhibits or blocks the binding of ASGR1 to a natural ligand thereof and/or the endocytosis of ASGR1; the monoclonal antibody comprises a light chain variable region and a heavy chain variable region, and is selected from any one of the following antibodies:
(a) the light chain variable region comprises LCDR1, LCDR2, and LCDR3 comprising the sequences as shown in SEQ ID NO: 4 to 6, respectively, or an equivalent of each; the heavy chain variable region comprises HCDR1, HCDR2, and HCDR3 comprising the sequences as shown in SEQ ID NO: 7 to 9, respectively, or an equivalent of each;
(b) the light chain variable region comprises LCDR1, LCDR2, and LCDR3 comprising the sequences as shown in SEQ ID NO: 10 to 12, respectively, or an equivalent of each; the heavy chain variable region comprises HCDR1, HCDR2, and HCDR3 comprising the sequences as shown in SEQ ID NO: 13 to 15, respectively, or an equivalent of each;
(c) the light chain variable region comprises LCDR1, LCDR2, and LCDR3 comprising the sequences as shown in SEQ ID NO: 16 to 18, respectively, or an equivalent of each; the heavy chain variable region comprises HCDR1, HCDR2, and HCDR3 comprising the sequences as shown in SEQ ID NO: 19 to 21, respectively, or an equivalent of each;
(d) the light chain variable region comprises LCDR1, LCDR2, and LCDR3 comprising the sequences as shown in SEQ ID NO: 22 to 24, respectively, or an equivalent of each; the heavy chain variable region comprises HCDR1, HCDR2, and HCDR3 comprising the sequences as shown in SEQ ID NO: 25 to 27, respectively, or an equivalent of each;
(e) the light chain variable region comprises the sequence as shown in SEQ ID NO: 28 or an equivalent thereof; the heavy chain variable region comprises the sequence as shown in SEQ ID NO: 29 or an equivalent thereof;
(f) the light chain variable region comprises the sequence as shown in SEQ ID NO: 30 or an equivalent thereof; the heavy chain variable region comprises the sequence as shown in SEQ ID NO: 31 or an equivalent thereof;
(g) the light chain variable region comprises the sequence as shown in SEQ ID NO: 32 or an equivalent thereof; the heavy chain variable region comprises the sequence as shown in SEQ ID NO: 33 or an equivalent thereof; and
(h) the light chain variable region comprises the sequence as shown in SEQ ID NO: 34 or an equivalent thereof; the heavy chain variable region comprises the sequence as shown in SEQ ID NO: 35 or an equivalent thereof.

In a second aspect, the present disclosure provides a pharmaceutical composition comprising any one of the monoclonal antibodies or the antigen-binding fragments thereof as described above, and a pharmaceutically acceptable carrier.

In a third aspect, the present disclosure provides a method for reducing the level of cholesterol and/or triglyceride in the blood or for treating and/or preventing a cardiovascular disease, comprising administering to a subject with a therapeutically effective amount of any one of the monoclonal antibodies or the antigen-binding fragments thereof as described above, or a pharmaceutical composition comprising any one of the monoclonal antibodies or the antigen-binding fragments thereof as described above.

In a fourth aspect, the present disclosure provides a use of the monoclonal antibody or the antigen-binding fragment thereof as described above or the pharmaceutical composition as described above in the manufacture of a medicament for reducing the level of cholesterol and/or triglyceride in the blood or for treating and/or preventing a cardiovascular disease.

In a fifth aspect, the present disclosure provides the monoclonal antibody or the antigen-binding fragment thereof as described above or the pharmaceutical composition as described above for use in reducing the level of cholesterol and/or triglyceride in the blood or in treating and/or preventing a cardiovascular disease.

In a sixth aspect, the present disclosure provides a nucleotide sequence encoding the monoclonal antibody or the antigen-binding fragment thereof according to the present disclosure. In another aspect, the present disclosure further provides a vector comprising the nucleotide sequence as described above. In another aspect, the present disclosure further provides a host cell comprising the vector as described above. In addition, the present disclosure further provides a cell line for producing the monoclonal antibody or the antigen-binding fragment thereof of the present disclosure, a recombinant expression vector comprising the nucleotide of the present disclosure, and a method for preparing the antibody by culturing an antibody-producing cell line.

In any one of the preceding aspects, the monoclonal antibody is preferably a humanized antibody or a chimeric antibody.

The inventor has also found that the combinational administration of an ASGR1 inhibitor with another lipid-lowering agent results in a significant synergistic lipid-lowering effect, with a significant reduction in the level of total cholesterol and triglyceride in the blood and/or liver.

In a seventh aspect, the present disclosure provides a pharmaceutical composition comprising a therapeutically effective amount of an ASGR1 inhibitor, a therapeutically effective amount of a second lipid-lowering agent, and a pharmaceutically acceptable carrier.

In an eighth aspect, the present disclosure provides a kit comprising a first pharmaceutical composition and a second pharmaceutical composition, wherein the first pharmaceutical composition comprises a therapeutically effective amount of an ASGR1 inhibitor and a pharmaceutically acceptable carrier; the second pharmaceutical composition comprises a therapeutically effective amount of a second lipid-lowering agent and a pharmaceutically acceptable carrier.

In a ninth aspect, the present disclosure provides a use of a combination of an ASGR1 inhibitor and a second lipid-lowering agent in the manufacture of a medicament for reducing the level of total cholesterol and/or triglyceride in the blood and/or liver or for treating and/or preventing a cardiovascular disease.

In a tenth aspect, the present disclosure provides a combination of an ASGR1 inhibitor and a second lipid-lowering agent for use in reducing the level of total cholesterol and/or triglyceride in the blood and/or liver or in treating and/or preventing a cardiovascular disease.

In an eleventh aspect, the present disclosure provides a method for reducing the level of total cholesterol and/or triglyceride in the blood and/or liver or for treating and/or preventing a cardiovascular disease, comprising administering to a subject with a therapeutically effective amount of an ASGR1 inhibitor and a therapeutically effective amount of a second lipid-lowering agent.

In any one of the preceding aspects, the ASGR1 inhibitor is preferably selected from an anti-ASGR1 monoclonal antibody or an antigen-binding fragment thereof, a nucleic acid targeting the coding nucleic acid of ASGR1, a nucleic acid aptamer targeting ASGR1, and a combination thereof.

In some embodiments, the ASGR1 monoclonal antibody binds to human ASGR1 comprising the sequence as shown in SEQ ID NO: 1 and inhibits or blocks the binding of human ASGR1 to a natural ligand thereof (e.g., asialoglycoprotein) and/or the endocytosis of human ASGR1. In some embodiments, the ASGR1 monoclonal antibody binds to a carbohydrate-binding region of ASGR1 and inhibits or blocks the binding of human ASGR1 to a natural ligand thereof (e.g., asialoglycoprotein) and/or the endocytosis of human ASGR1. In some embodiments, the carbohydrate-binding region of ASGR1 comprises, or consists essentially of, or consists of the sequence of SEQ ID NO: 2. In some embodiments, the carbohydrate-binding region of ASGR1 comprises, or consists essentially of, or consists of the sequence of SEQ ID NO: 3. In some embodiments, the ASGR1 monoclonal antibody binds to an epitope comprising one or more of Q240, D242, W244, E253, N265, D266, D267, R237, N209, H257, T259, and Y273 in SEQ ID NO: 1. In some embodiments, the ASGR1 monoclonal antibody binds to an epitope comprising one or more of Q240, D242, W244, E253, N265, and D266 in SEQ ID NO: 1. In some embodiments, the ASGR1 monoclonal antibody binds to an epitope comprising Q240, D242, W244, E253, N265, and D266 in SEQ ID NO: 1.

In some embodiments, the anti-ASGR1 monoclonal antibody or the antigen-binding fragment thereof is the monoclonal antibody or the antigen-binding fragment thereof according to the first aspect.

In any one of the preceding aspects, the second lipid-lowering agent is preferably an HMGCR inhibitor, an ATP-citrate lyase (ACL) inhibitor, a NPC1L1 inhibitor, and/or a PCSK9 inhibitor. In some embodiments, the HMGCR inhibitor is a statin. In some embodiments, the statin is selected from lovastatin, simvastatin, pravastatin, fluvastatin, atorvastatin, rosuvastatin and pitavastatin. In some embodiments, the statin is atorvastatin. In some embodiments, the ACL inhibitor is bempedoic acid. In some embodiments, the NPC1L1 inhibitor is Ezetimibe. In some embodiments, the PCSK9 inhibitor is an anti-PCSK9 antibody or an interfering nucleic acid (e.g., siRNA or shRNA) targeting the PCSK9-coding gene. In some embodiments, the PCSK9 inhibitor is Evolocumab, Alirocumab, or Inclisiran.

Other aspects and advantages of the present disclosure may be apparently obtained from the following detailed description of the present disclosure.

### BRIEF DESCRIPTION OF THE DRAWINGS

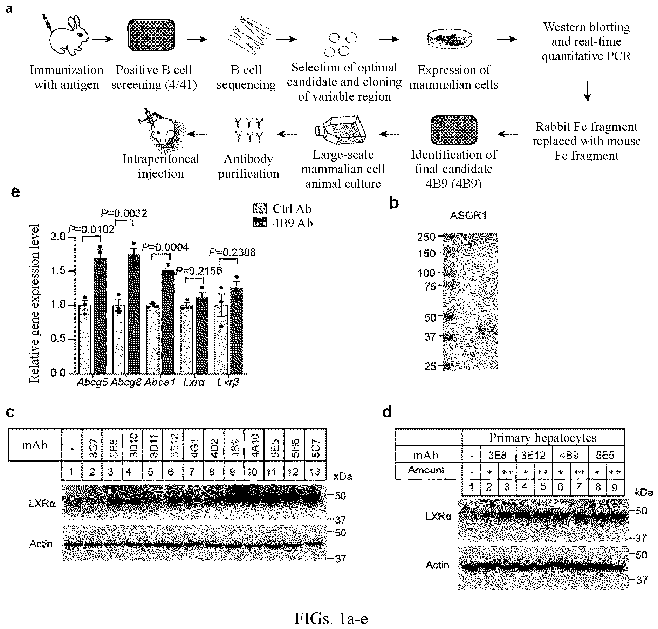

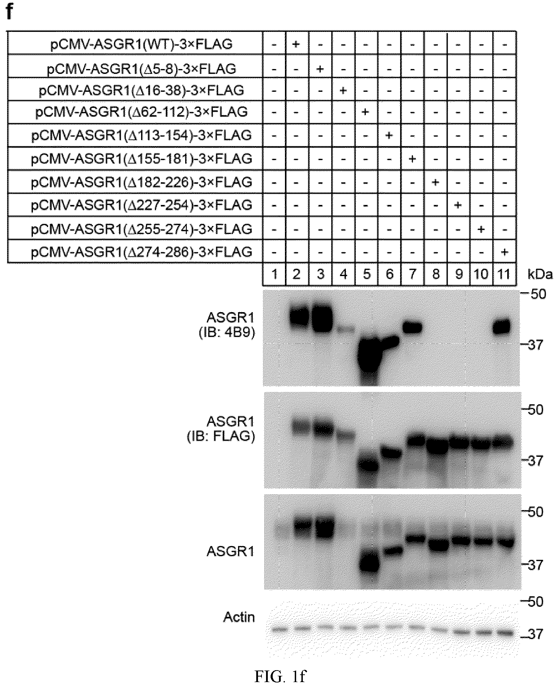

**FIG. 1** **shows the preparation of ASGR1 neutralizing antibody.** (a) Steps for preparing ASGR1 monoclonal neutralizing antibody. Purified ASGR1 protein is used as the antigen to immunize rabbits, and after the first round of screening, four B-cell monoclonal antibodies are selected as candidates. The coding region of the variable region of the antibody is then sequenced and cloned into an antibody expression vector, which is transfected into mammalian cells. The efficiency of transfection is confirmed by Western blotting and real-time quantitative PCR. Finally, the rabbit-derived Fc fragment is replaced with a murine Fc fragment. The neutralizing antibody 4B9 is selected for large-scale production and used in subsequent experiments. (b) ASGR1 protein purified from HEK293T cells is stained with Coomassie Brilliant Blue. (c) Huh7 cells are incubated with purified neutralizing antibody for 72 hours, followed by Western blotting analysis of the protein expression of LXRα. (d) Primary hepatocytes are isolated from 8-week-old wild-type C57B/L6 mice. The primary hepatocytes are incubated with different monoclonal neutralizing antibodies at various concentrations for 72 hours, after which cells are collected, lysed, and subjected to Western blotting analysis. (e) Primary hepatocytes are isolated from 8-week-old wild-type C57B/L6 mice. The primary hepatocytes are incubated with ASGR1 neutralizing antibody 4B9 for 72 hours, after which cells are collected, total RNA is extracted from the cells, and target genes of LXR are analyzed by real-time quantitative PCR. (f) Huh7 cells are transfected with a specified plasmid. After 48 hours of transfection, cells are collected and lysed, followed by Western blotting analysis of the expression of various proteins.

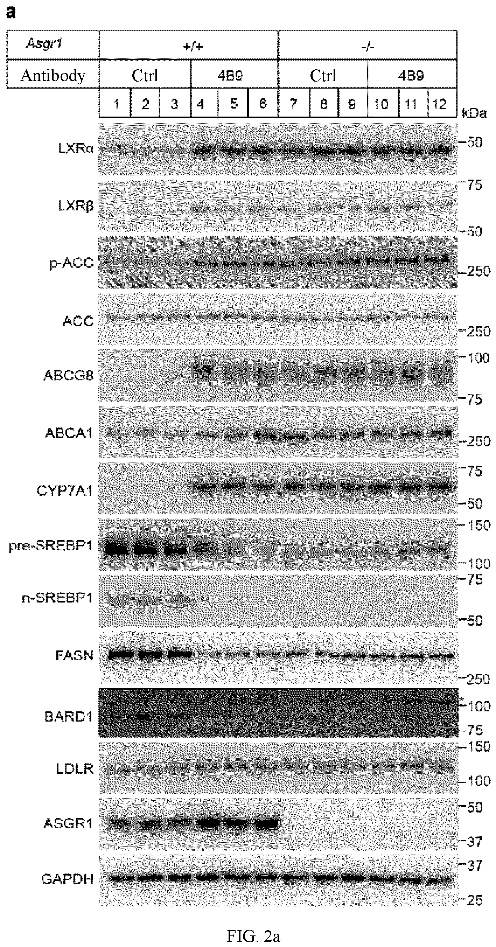

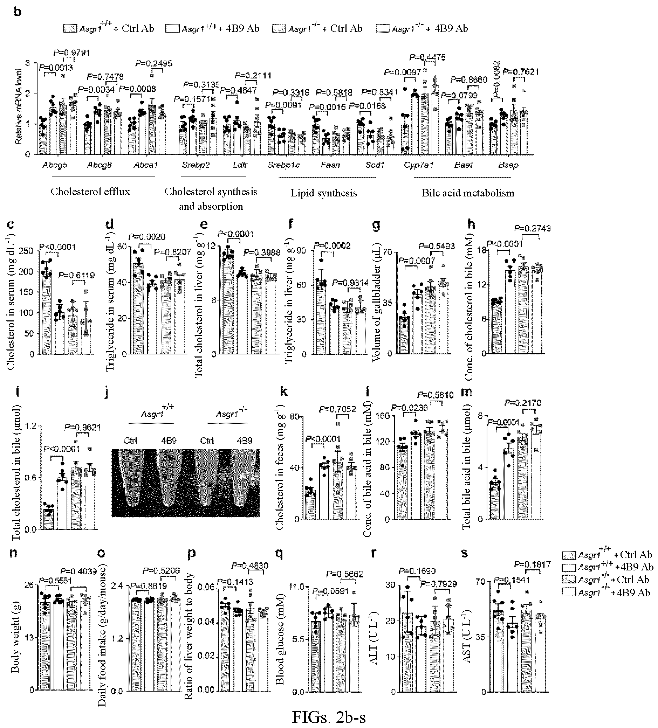

**FIG. 2** **shows an increase in cholesterol efflux and a reduction in blood lipid and liver lipid by ASGR1 neutralizing antibody.** 8-week-old *Asgr1* knockout mice and wild-type littermates are randomly divided into 4 groups as shown in the figure according to the genotype, with 6 mice per group. The mice are given free access to water and fed a high-fat, high-cholesterol, and cholate (HF/HC/BS) diet (60% fat, 1.25% cholesterol, and 0.5% cholate). Meanwhile, the mice are intraperitoneally injected every other day with control antibody or ASGR1 neutralizing antibody 4B9 at a dose of 10 mg/kg/day. After 14 days, the mice are fasted for 4 hours before being sacrificed. All data are presented as mean ± SEM. Statistical significance is calculated using an unpaired two-tailed Student's t-test. *p<0.05, **p<0.01, ***p<0.001. (a) Western blotting analysis of liver samples; (b) real-time quantitative PCR analysis of genes related to cholesterol efflux, cholesterol synthesis and absorption, lipid synthesis, and bile acid metabolism in the liver of mice, with cyclophilin as an internal reference; (c) total cholesterol in serum; (d) triglyceride in serum; (e) total cholesterol in liver; (f) triglyceride in liver; (g) volume of gallbladder; (h) concentration of cholesterol in bile; (i) total cholesterol in bile; (j) representative picture of volume of gallbladder; (k) total cholesterol in feces; (l) concentration of bile acid in bile; (m) total bile acid in bile; (n) body weight; (o) daily food intake; (p) ratio of liver weight to body weight; (q) blood glucose; (r) alanine transaminase in serum; (s) aspartate transaminase in serum.

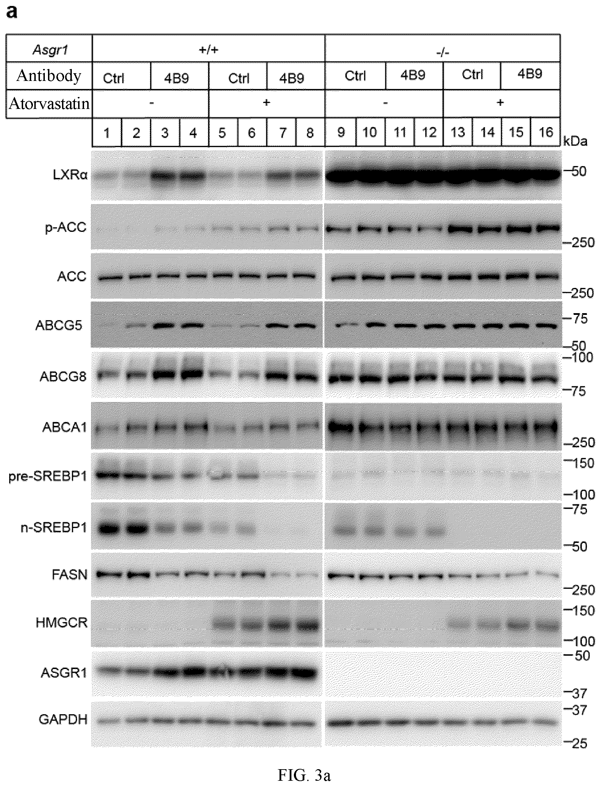

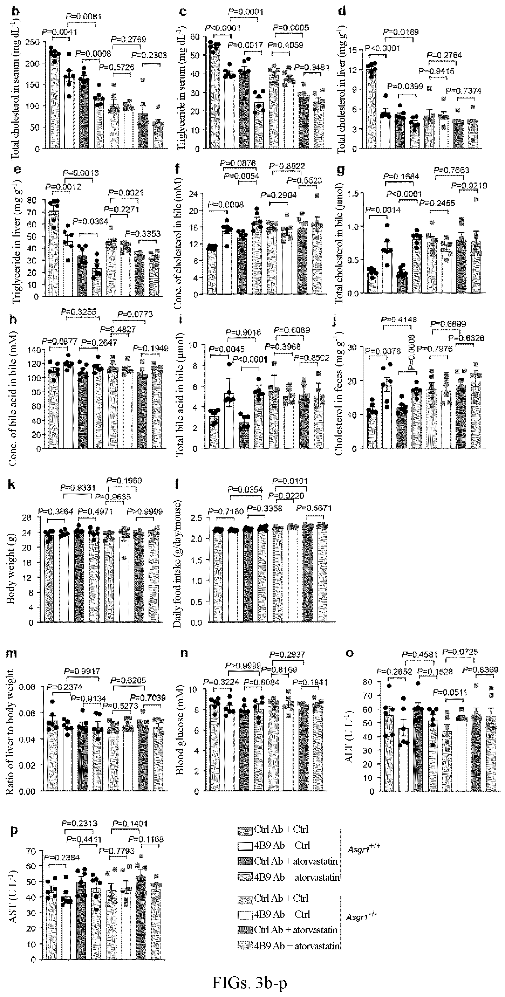

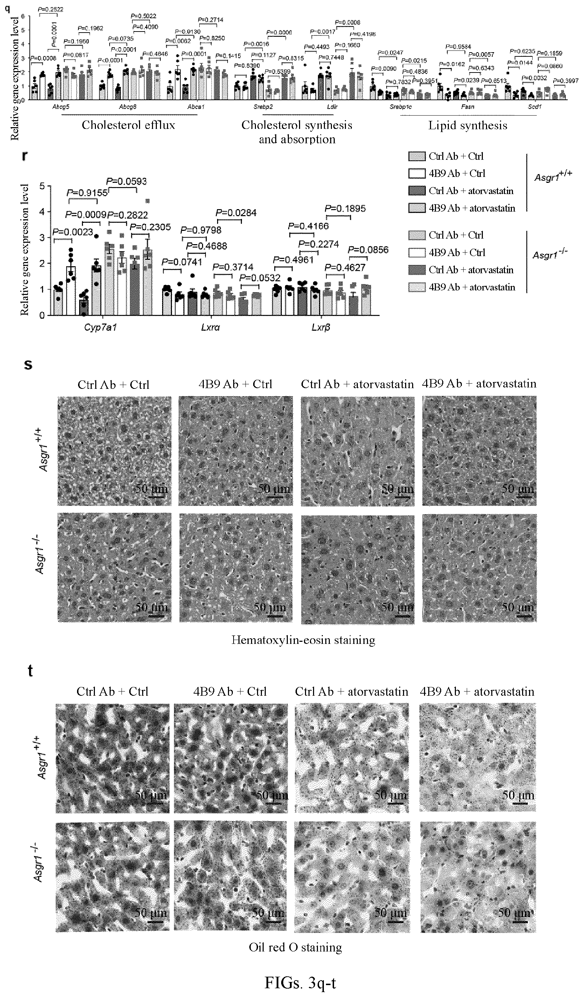

**FIG. 3** **shows the synergistic lipid-lowering effect by a combination of ASGR1 neutralizing antibody and atorvastatin.** 8-week-old *Asgr1* knockout mice and wild-type littermates are randomly divided into 8 groups as shown in the figure according to the genotype, with 6 mice per group. The mice are given free access to water and fed a high-fat, high-cholesterol, and cholate (HF/HC/BS) diet (60% fat, 1.25% cholesterol, and 0.5% cholate). Meanwhile, the mice are intraperitoneally injected every other day with control antibody or ASGR1 neutralizing antibody 4B9 at a dose of 10 mg/kg/day and are intragastrically administered each day with atorvastatin at a dose of 30 mg/kg/day. After 14 days, the mice are fasted for 4 hours before being sacrificed. All data are presented as mean ± SEM. Statistical significance is calculated using an unpaired two-tailed Student's t-test. *p<0.05, **p<0.01, ***p<0.001. (a) Western blotting analysis of liver samples; (b) total cholesterol in serum; (c) triglyceride in serum; (d) total cholesterol in liver; (e) triglyceride in liver; (f) concentration of cholesterol in bile; (g) total cholesterol in bile; (h) concentration of bile acid in bile; (i) total bile acid in bile; (j) total cholesterol in feces; (k) body weight; (l) daily food intake; (m) ratio of liver weight to body weight; (n) blood glucose; (o) alanine transaminase in serum; (p) aspartate transaminase in serum; (q-r) real-time quantitative PCR analysis of genes related to cholesterol efflux, cholesterol synthesis and absorption, lipid synthesis, and bile acid synthesis in the liver of mice, and genes in other pathways, with cyclophilin as an internal reference; (s) hematoxylin-eosin staining of liver sections; (t) oil red O staining of liver sections.

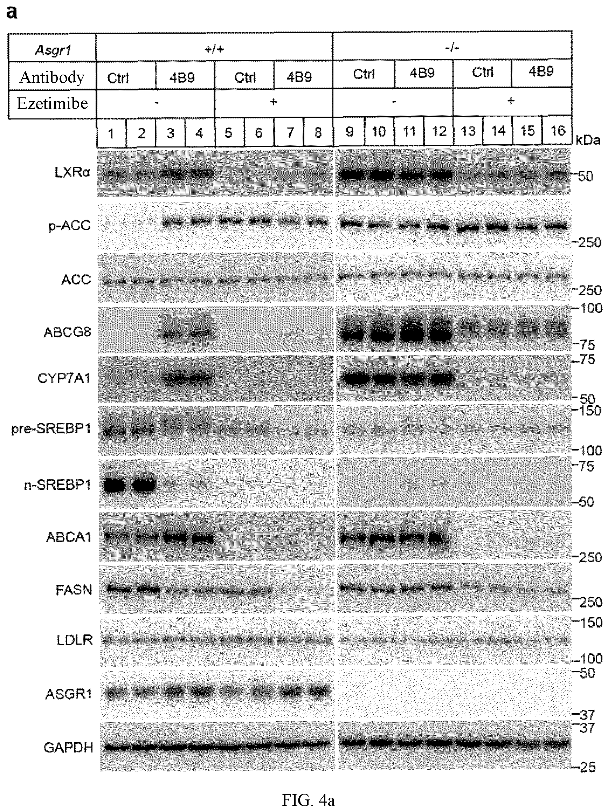

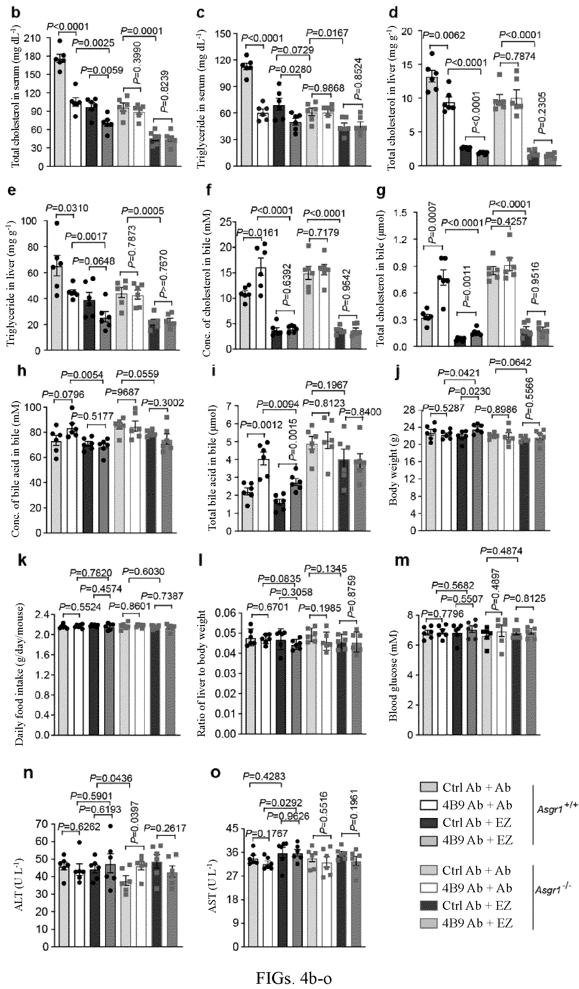

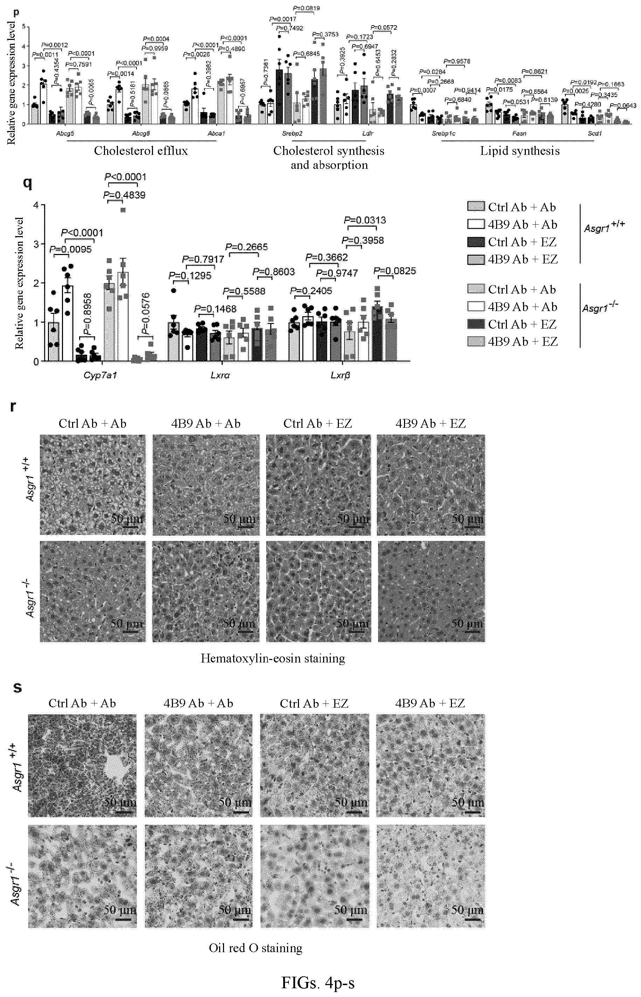

**FIG. 4** **shows the synergistic effect by a combination of neutralizing antibody 4B9 and Ezetimibe.** 8-week-old *Asgr1* knockout mice and wild-type littermates are randomly divided into 8 groups as shown in the figure according to the genotype, with 6 mice per group. The mice are given free access to water and fed a high-fat, high-cholesterol, and cholate (HF/HC/BS) diet (60% fat, 1.25% cholesterol, and 0.5% cholate). Meanwhile, the mice are intraperitoneally injected every other day with control antibody or ASGR1 neutralizing antibody 4B9 at a dose of 10 mg/kg/day and are intragastrically administered each day with Ezetimibe at a dose of 10 mg/kg/day. After 8 days, the mice are fasted for 4 hours before being sacrificed. All data are presented as mean ± SEM. Statistical significance is calculated using an unpaired two-tailed Student's t-test. *p<0.05, **p<0.01, ***p<0.001. (a) Western blotting analysis of liver samples; (b) total cholesterol in serum; (c) triglyceride in serum; (d) total cholesterol in liver; (e) triglyceride in liver; (f) concentration of cholesterol in bile; (g) total cholesterol in bile; (h) concentration of bile acid in bile; (i) total bile acid in bile; (j) body weight; (k) daily food intake; (l) ratio of liver weight to body weight; (m) blood glucose; (n) alanine transaminase in serum; (o) aspartate transaminase in serum; (p-q) real-time quantitative PCR analysis of genes related to cholesterol efflux, cholesterol synthesis and absorption, lipid synthesis, and bile acid synthesis in the liver of mice, and genes in other pathways, with cyclophilin as an internal reference; (r) hematoxylin-eosin staining of liver sections; (s) oil red O staining of liver sections.

### DETAILED DESCRIPTION OF THE PREFERRED EMBODIMENT

### Definition

In the present disclosure, "about" refers to a value that is within an acceptable error range for a particular value as determined by one of ordinary skill in the art, which will depend in part on how the value is measured or determined *(i.e.,* the limitations of the measurement system). For example, "about" may mean a standard deviation within or above 1 per the practice in the art. Alternatively, "about" or "comprising essentially" may mean a range of up to 20%. Furthermore, the term may mean up to an order of magnitude or up to 5-fold of a value, particularly with respect to biological systems or processes. When a particular value is provided in the present disclosure and claims, unless otherwise specified, the meaning of "about" or "comprising essentially" should be assumed to be within an acceptable error range for the particular value.

The composition or method described herein as "comprising" one or more elements or steps are open, meaning that the elements or steps are necessary, but other elements or steps may be added within the scope of the composition or method. It should also be understood that any composition or method described as "comprising" one or more elements or steps also describes a corresponding, more limited, composition or method "consisting essentially of the elements or steps", meaning that the composition or method includes the necessary elements or steps, and may also include additional elements or steps that do not substantially affect the basic and novel features of the composition or method.

As used herein, the term "antibody" refers to any form of an antibody that exhibits the desired biological activity (*e.g*., inhibiting the binding of a ligand to its receptor, or by inhibiting ligand-induced receptor signaling). "Antibody fragment" and "antigen-binding fragment" refer to an antigen-binding fragment of an antibody and an antibody analog, typically including at least a portion of the antigen-binding region or variable region (*e.g.,* one or more CDRs) of the parental antibody. In some embodiments, the antibody is a monoclonal antibody. In some other embodiments, the antibody is a polyclonal antibody.

As used herein, the term "monoclonal antibody" refers to an antibody obtained from a population of substantially homogeneous antibodies, *i.e.,* the individual antibodies that make up the population are identical except for possible naturally occurring mutations that may be present in small amounts. Monoclonal antibodies are highly specific and can be directed to a single antigenic site. Furthermore, each monoclonal antibody is only directed to a single determinant on an antigen, as opposed to conventional (polyclonal) antibody preparations that typically include different antibodies directed to different determinants (epitopes). The modifier "monoclonal" refers to the characteristic of an antibody obtained from a population of substantially homogeneous antibodies, and is not to be construed as requiring production of the antibody by any particular method. For example, monoclonal antibodies used in the present disclosure can be prepared by hybridoma or recombinant DNA methods.

The monoclonal antibody may include a "chimeric" antibody, a humanized antibody, or a fully humanized antibody. In some embodiments, the antibody forms part of a larger biomolecule, such as a fusion protein or antibody-drug conjugate. The antibody fragment retains at least some of the binding specificity of the parental antibody. Typically, the antibody fragment retains at least 10% of the parental binding activity when the activity is expressed on a molar basis. Preferably, the antibody fragment retains at least 20%, 50%, 70%, 80%, 90%, 95% or 100% or more of the binding affinity of the parental antibody for the target.

Thus, as used herein, examples of antibody fragments include, but are not limited to: Fab, Fab', F(ab')₂, and Fv fragments; diabodies; linear antibodies; single-chain antibody molecules, such as sc-Fv; nanobodies; domain antibodies; and multispecific antibodies (*e.g.,* bispecific antibodies) formed from antibody fragments, CAR-T, and BiTE, etc. A "Fab fragment" consists of one light chain and the CH1 and variable region of one heavy chain. The heavy chain of a Fab molecule cannot form a disulfide bond with another heavy chain molecule. A "Fab' fragment" contains one light chain and a portion of one heavy chain comprising the VH domain and the CH1 domain as well as the region between the CH1 and CH2 domains, such that an interchain disulfide bond can be formed between the two heavy chains of two Fab' fragments to form a F(ab')₂ molecule. A "F(ab')₂ fragment" contains two light chains and two heavy chains comprising a portion of the constant region between the CH1 and CH2 domains, such that an interchain disulfide bond is formed between the two heavy chains. Thus, the F(ab')₂ fragment consists of two Fab' fragments held together by a disulfide bond between the two heavy chains. A "Fv region" comprises variable regions from both the heavy and light chains, but lacks the constant region. A "single-chain Fv antibody" (or "scFv antibody") refers to an antibody fragment comprising the VH and VL domains of an antibody, wherein the domains are present in a single polypeptide chain. Generally, the Fv polypeptide further comprises a polypeptide linker between the VH and VL domains which enables the scFv to form the desired structure for antigen binding.

"Fc" or "Fc segment" or "Fc region" refers to a polypeptide comprising the constant region of an antibody excluding the first constant region immunoglobulin domain, and in some cases, excluding part of the hinge. Thus, Fc refers to the last two constant region immunoglobulin domains of IgA, IgD and IgG, the last three constant region immunoglobulin domains of IgE and IgM, and the flexible hinge N-terminus to these domains. In some embodiments, amino acid modifications are made to the Fc region, for example to alter the binding to one or more FcγR receptors or FcRn receptors.

As used herein, the term "epitope" refers to a portion of an antigen to which an antibody or antigen-binding fragment binds. In some embodiments, the epitope may be a conformational epitope, *i.e.,* portions of an antigen that are not covalently contiguous in the antigen but are in close proximity to each other in three-dimensional space when the antigen is in a relevant conformation. For example, for ASGR1, the conformational epitope is an epitope comprised of a plurality of amino acid residues that are not contiguous in the extracellular domain of ASGR1. In some embodiments, the epitope may be a linear epitope, *i.e.,* an epitope comprising a sequence of amino acid residues contiguous in the primary structure in the extracellular domain of ASGR1. Methods for determining the exact sequence and/or in particular amino acid residues of an epitope of ASGR1 are known in the literature, including competing with peptides from the antigen sequence for binding to ASGR1 sequences from different species, truncation and/or mutagenesis (*e.g.,* by alanine scanning or other site-directed mutagenesis), phage display-based screening, or (co-)crystallography techniques.

As used herein, the term "humanized antibody" refers to an antibody comprising the CDR of an antibody derived from mammals other than humans, and the framework region (FR) and constant region of a human antibody.

As used herein, the term "chimeric antibody" shall be taken to refer to any antibody in which the immunoreactive region or site is obtained or derived from a first species and the constant region (which may be intact, partial, or modified in accordance with the present disclosure) is obtained from a second species. In certain embodiments, the target binding region or site will be from a non-human source (*e.g.,* mouse or primate) and the constant region is from a human source.

An "equivalent" of an antibody or polypeptide refers to an antibody or polypeptide having a certain homology or sequence identity to the amino acid sequence of the antibody or polypeptide. In some aspects, the sequence identity is at least about 70%, 75%, 80%, 85%, 90%, 95%, 98%, or 99%. In some aspects, compared to a reference antibody or polypeptide, the equivalent thereof has one, two, three, four, or five additions, deletions, substitutions, and combinations thereof. In some aspects, the equivalent of an antibody or polypeptide retains the activity (*e.g.,* epitope binding) or structure (*e.g.,* salt bridge) of the reference sequence.

As used herein, a "variant" of a sequence refers to a sequence that differs from the sequence shown at one or more amino acid residues but retains the biological activity of the resulting molecule.

As used herein, "% identity" between two sequences refers to a function of the number of identical positions shared by the sequences (*i.e.,* % homology = number of identical positions/total number of positions × 100), taking into account the number of gaps and the length of each gap, which needs to be introduced for optimal alignment of the two sequences. The comparison of sequences and determination of % identity between two sequences can be accomplished using a mathematical algorithm.

"Conservative substitution" refers to an amino acid substitution known to those skilled in the art, which is generally made without altering the biological activity of the resulting molecule. Generally, it is recognized by those skilled in the art that single amino acid substitutions in non-essential regions of a polypeptide do not or do not substantially alter the biological activity. "Do not or do not substantially alter" means that one or more aspects have no more than about 20%, about 15%, about 10%, about 9%, about 8%, about 7%, about 6%, about 5%, about 4%, about 3%, about 2%, or about 1% difference as compared to the compared subject when measured in the same manner or a similar manner.

Typically, HCDR3 and LCDR3 are considered to play a more important role in antigen recognition than the other CDRs. Thus, in the case of substitutions, conservative substitutions are preferably made to the CDRs other than HCDR3 and LCDR3 in the present disclosure. In some embodiments, HCDR1, HCDR3, and LCDR3 are not substituted. Preferred amino acid substitutions include, but are not limited to: substitutions that (1) reduce susceptibility to proteolysis, (2) reduce susceptibility to oxidation, (3) alter binding affinity to form a protein complex, (4) provide or modify other physicochemical or functional properties of these analogs. Analogs may include various mutations of sequences other than naturally occurring peptide sequences. For example, single or multiple amino acid substitutions (preferably conservative amino acid substitutions) can be made in naturally occurring sequences (preferably in a portion of the polypeptide outside the regions forming intermolecular contacts). Conservative amino acid substitutions should not substantially alter the structural features of a parental sequence (e.g., amino acid substitutions should not tend to disrupt a helix present in the parental sequence, or characterize other secondary structure types that disrupt the parental sequence).

It is contemplated that the binding domain of the monoclonal antibody or antigen-binding fragment thereof of the present disclosure may carry a signal peptide, which is typically located at the N-terminus of the secretory protein and generally consists of 15 to 30 amino acids. After the signal peptide sequence is synthesized, it is recognized by the signal recognition particle (SRP), suspending or slowing down protein synthesis. The signal recognition particle carries the ribosome to the endoplasmic reticulum, where protein synthesis resumes. Under the guidance of the signal peptide, the newly synthesized protein enters the lumen of the endoplasmic reticulum, and the signal peptide sequence is cleaved off by the action of a signal peptidase. If a stop-transfer sequence is present at the C-terminus of the nascent peptide chain, it may not be cleaved off by the signal peptidase; for example, ovalbumin contains an internal signal peptide. Neither its precursor nor mature form undergoes cleavage by the peptidase.

"Specific" binding, when referring to ligand/receptor, antibody/antigen, or other binding pairs, refers to a binding reaction that determines the presence of the protein in a heterogeneous population of proteins and/or other biological agents. Thus, under specified conditions, a particular ligand/antigen binds to a particular receptor/antibody and does not bind to other proteins present in the sample in a significant amount. "Specific binding" means that the monoclonal antibody or antigen-binding fragment thereof of the present disclosure is capable of specifically interacting with at least two, three, four, five, six, seven, eight, or more amino acids of each human target molecule. "Specific binding" of an antibody is characterized primarily by two parameters: a qualitative parameter (the binding epitope or where the antibody binds) and a quantitative parameter (the binding affinity or binding strength). The binding epitope of an antibody can be determined by FACS, peptide-spot epitope mapping, mass spectrometry, or peptide ELISA. The binding strength of an antibody to a particular epitope can be determined by Biacore and/or ELISA. Signal-to-noise ratio is typically used as a representative method for measuring and calculating binding specificity. In such a signal-to-noise ratio, the signal represents the strength of binding of the antibody to the target epitope, while the noise represents the strength of binding of the antibody to other non-target epitopes. Preferably, the antibody evaluated can be considered to bind to the target epitope in a specific manner, *i.e.,* "specific binding", when the signal-to-noise ratio for the target epitope is about 50. An antigen-binding protein (including antibodies) "specifically binds" to an antigen if the antigen-binding protein (including antibodies) binds to the antigen with a high binding affinity as determined by the affinity constant (KD) value. In some embodiments, the affinity constant KD is less than 10⁻⁹ M. As used herein, the term "KD" refers to the affinity constant for a particular antibody-antigen interaction.

As used herein, the term "patient" or "subject" refers to any organism to which the provided antibody, the antigen-binding fragment thereof, or the pharmaceutical composition is administered or can be administered for experimental, diagnostic, prophylactic, cosmetic, and/or therapeutic purposes. Typical subjects include animals (*e.g.,* mammals, such as mice, rats, rabbits, non-human primates, and/or humans). In some embodiments, the subject is a human. In some embodiments, the subject is suffering from or susceptible to one or more disorders or conditions. The patient may exhibit one or more symptoms of a disorder or condition, or may have been diagnosed with one or more disorders or conditions. In some embodiments, the patient is receiving or has received some therapy for diagnosing and/or treating such a disease, disorder, or condition.

In the present disclosure, the term "treatment" refers to a therapeutic and prophylactic measure that prevents or slows the occurrence of an undesirable physiological change or the development or progression of a condition in a subject. The advantageous or desirable clinical effects include, but are not limited to, relief of symptoms, reduction in the severity of a disease, stabilization (*i.e.,* no worsening) of disease state, delay or slowing of disease progression, alleviation or remission of disease state, and partial or complete cure of a disease, regardless of whether or not the above effects are detectable. "Treatment" may also refer to prolonged survival compared to no treatment. Subjects in need of treatment includes those who have suffered from the disease or condition, and those who are at risk of the disease or condition, or those who are to be prevented from the disease or condition.

"Administration" and "treatment", when referring to an animal, human, experimental subject, cell, tissue, organ, or biological fluid, refer to contacting an exogenous drug, therapeutic agent, diagnostic agent, or composition with the animal, human, subject, cell, tissue, organ, or biological fluid. "Administration" and "treatment" may refer to, for example, therapeutic, pharmacokinetic, diagnostic, research, and experimental methods. Treatment of a cell encompasses contacting a reagent with the cell as well as contacting a reagent with a fluid, wherein the fluid is in contact with the cell. "Administration" and "treatment" also mean *in vitro* and *ex vivo* treatment of a cell, *e.g.,* by a reagent, a diagnostic agent, or a binding composition, or by another cell.

As used herein, the term "therapeutically effective amount" or "effective amount" refers to an amount of the monoclonal antibody binding to ASGR1 or the antigen-binding fragment thereof of the present disclosure that, when administered alone or in combination with an additional therapeutic agent to a cell, tissue, or subject, is effective in preventing or ameliorating the disease or disorder to be treated. The therapeutically effective amount further refers to an amount of the compound that is sufficient to cause amelioration of symptoms, such as treatment, healing, prevention, or amelioration of a related medical condition, or an increase in the rate of treatment, healing, prevention, or amelioration of the condition. When an individual is administered an active ingredient alone, the therapeutically effective amount refers to the ingredient alone. When a combination is administered, the therapeutically effective amount refers to a combined amount of the active ingredient that produces the therapeutic effect, whether administered in combination, sequentially, or simultaneously. The therapeutically effective amount will alleviate the symptoms generally by at least 10%; typically by at least 20%; preferably by at least about 30%; more preferably by at least 40%, and most preferably by at least 50%.

As used herein, a "pharmaceutically acceptable carrier" includes a material that, when combined with an active ingredient of a composition, allows the ingredient to retain the biological activity without causing a destructive reaction with the immune system of the subject. These carriers may include stabilizers, preservatives, salt or sugar complexes, or crystals. "Pharmaceutically acceptable" refers to a molecule and component that does not produce an allergic reaction or a similar undesired reaction when administered to a human body.

### ASGR1 monoclonal antibody

In one aspect of the present disclosure, the present disclosure provides a monoclonal antibody or an antigen-binding fragment thereof that binds to ASGR1, wherein the monoclonal antibody inhibits or blocks the binding of ASGR1 to a natural ligand thereof and/or the endocytosis of ASGR1.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 4 to 6, respectively, or an equivalent of each; the heavy chain variable region comprises HCDR1, HCDR2, and HCDR3 comprising the sequences as shown in SEQ ID NO: 7 to 9, respectively, or an equivalent of each.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 10 to 12, respectively, or an equivalent of each; the heavy chain variable region comprises HCDR1, HCDR2, and HCDR3 comprising the sequences as shown in SEQ ID NO: 13 to 15, respectively, or an equivalent of each.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 16 to 18, respectively, or an equivalent of each; the heavy chain variable region comprises HCDR1, HCDR2, and HCDR3 comprising the sequences as shown in SEQ ID NO: 19 to 21, respectively, or an equivalent of each.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 22 to 24, respectively, or an equivalent of each; the heavy chain variable region comprises HCDR1, HCDR2, and HCDR3 comprising the sequences as shown in SEQ ID NO: 25 to 27, respectively, or an equivalent of each.

In some embodiments, the ASGR1 monoclonal antibody comprises a light chain variable region and a heavy chain variable region; the light chain variable region comprises the sequence as shown in SEQ ID NO: 28 or an equivalent thereof; the heavy chain variable region comprises the sequence as shown in SEQ ID NO: 29 or an equivalent thereof.

In some embodiments, the ASGR1 monoclonal antibody comprises a light chain variable region and a heavy chain variable region; the light chain variable region comprises the sequence as shown in SEQ ID NO: 30 or an equivalent thereof; the heavy chain variable region comprises the sequence as shown in SEQ ID NO: 31 or an equivalent thereof.

In some embodiments, the ASGR1 monoclonal antibody comprises a light chain variable region and a heavy chain variable region; the light chain variable region comprises the sequence as shown in SEQ ID NO: 32 or an equivalent thereof; the heavy chain variable region comprises the sequence as shown in SEQ ID NO: 33 or an equivalent thereof.

In some embodiments, the ASGR1 monoclonal antibody comprises a light chain variable region and a heavy chain variable region; the light chain variable region comprises the sequence as shown in SEQ ID NO: 34 or an equivalent thereof; the heavy chain variable region comprises the sequence as shown in SEQ ID NO: 35 or an equivalent thereof.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 4 to 6, respectively, or a variant of each, wherein the variant has a single substitution, deletion, or insertion; the heavy chain variable region comprises HCDR1, HCDR2, and HCDR3 comprising the sequences as shown in SEQ ID NO: 7 to 9, respectively, or a variant of each, wherein the variant has a single substitution, deletion, or insertion.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 10 to 12, respectively, or a variant of each, wherein the variant has a single substitution, deletion, or insertion; the heavy chain variable region comprises HCDR1, HCDR2, and HCDR3 comprising the sequences as shown in SEQ ID NO: 13 to 15, respectively, or a variant of each, wherein the variant has a single substitution, deletion, or insertion.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 16 to 18, respectively, or a variant of each, wherein the variant has a single substitution, deletion, or insertion; the heavy chain variable region comprises HCDR1, HCDR2, and HCDR3 comprising the sequences as shown in SEQ ID NO: 19 to 21, respectively, or a variant of each, wherein the variant has a single substitution, deletion, or insertion.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 22 to 24, respectively, or a variant of each, wherein the variant has a single substitution, deletion, or insertion; the heavy chain variable region comprises HCDR1, HCDR2, and HCDR3 comprising the sequences as shown in SEQ ID NO: 25 to 27, respectively, or a variant of each, wherein the variant has a single substitution, deletion, or insertion.

In some embodiments, the ASGR1 monoclonal antibody comprises a light chain variable region and a heavy chain variable region; the light chain variable region comprises the sequence as shown in SEQ ID NO: 28 or a variant thereof having at least 75%, 80%, 85%, 90%, 95%, 98%, or 99% sequence identity to SEQ ID NO: 28; the heavy chain variable region comprises the sequence as shown in SEQ ID NO: 29 or a variant thereof having at least 75%, 80%, 85%, 90%, 95%, 98%, or 99% sequence identity to SEQ ID NO: 29.

In some embodiments, the ASGR1 monoclonal antibody comprises a light chain variable region and a heavy chain variable region; the light chain variable region comprises the sequence as shown in SEQ ID NO: 30 or a variant thereof having at least 75%, 80%, 85%, 90%, 95%, 98%, or 99% sequence identity to SEQ ID NO: 30; the heavy chain variable region comprises the sequence as shown in SEQ ID NO: 31 or a variant thereof having at least 75%, 80%, 85%, 90%, 95%, 98%, or 99% sequence identity to SEQ ID NO: 31.

In some embodiments, the ASGR1 monoclonal antibody comprises a light chain variable region and a heavy chain variable region; the light chain variable region comprises the sequence as shown in SEQ ID NO: 32 or a variant thereof having at least 75%, 80%, 85%, 90%, 95%, 98%, or 99% sequence identity to SEQ ID NO: 32; the heavy chain variable region comprises the sequence as shown in SEQ ID NO: 33 or a variant thereof having at least 75%, 80%, 85%, 90%, 95%, 98%, or 99% sequence identity to SEQ ID NO: 33.

In some embodiments, the ASGR1 monoclonal antibody comprises a light chain variable region and a heavy chain variable region; the light chain variable region comprises the sequence as shown in SEQ ID NO: 34 or a variant thereof having at least 75%, 80%, 85%, 90%, 95%, 98%, or 99% sequence identity to SEQ ID NO: 34; the heavy chain variable region comprises the sequence as shown in SEQ ID NO: 35 or a variant thereof having at least 75%, 80%, 85%, 90%, 95%, 98%, or 99% sequence identity to SEQ ID NO: 35.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 4 to 6, respectively; the heavy chain variable region comprises HCDR1, HCDR2, and HCDR3 comprising the sequences as shown in SEQ ID NO: 7 to 9, respectively.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 10 to 12, respectively; the heavy chain variable region comprises HCDR1, HCDR2, and HCDR3 comprising the sequences as shown in SEQ ID NO: 13 to 15, respectively.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 16 to 18, respectively; the heavy chain variable region comprises HCDR1, HCDR2, and HCDR3 comprising the sequences as shown in SEQ ID NO: 19 to 21, respectively.

In some embodiments, the ASGR1 monoclonal antibody comprises a light chain variable region and a heavy chain variable region; the light chain variable region comprises LCDR1, LCDR2, and LCDR3 comprising the sequences as shown in SEQ ID NO: 22 to 24, respectively; the heavy chain variable region comprises HCDR1, HCDR2, and HCDR3 comprising the sequences as shown in SEQ ID NO: 25 to 27, respectively.

In any one of the above embodiments, the LCDR1 to LCDR3 and HCDR1 to HCDR3 are defined based on any one of the IMGT, Kabat, Chothia, Contact, or Martin definition schemes. In some embodiments, the LCDR1 to LCDR3 and HCDR1 to HCDR3 are defined based on the IMGT definition scheme.

In some embodiments, the ASGR1 monoclonal antibody comprises a light chain variable region and a heavy chain variable region; the light chain variable region comprises the sequence as shown in SEQ ID NO: 28; the heavy chain variable region comprises the sequence as shown in SEQ ID NO: 29.

In some embodiments, the ASGR1 monoclonal antibody comprises a light chain variable region and a heavy chain variable region; the light chain variable region comprises the sequence as shown in SEQ ID NO: 30; the heavy chain variable region comprises the sequence as shown in SEQ ID NO: 31.

In some embodiments, the ASGR1 monoclonal antibody comprises a light chain variable region and a heavy chain variable region; the light chain variable region comprises the sequence as shown in SEQ ID NO: 32; the heavy chain variable region comprises the sequence as shown in SEQ ID NO: 33.

In some embodiments, the ASGR1 monoclonal antibody comprises a light chain variable region and a heavy chain variable region; the light chain variable region comprises the sequence as shown in SEQ ID NO: 34; the heavy chain variable region comprises the sequence as shown in SEQ ID NO: 35.

In some embodiments, the ASGR1 monoclonal antibody is of an IgG type, such as an IgG1, IgG2, IgG3, or IgG4 type. In some embodiments, the ASGR1 monoclonal antibody is of the IgG1 type.

In some embodiments, the substitutions described herein are conservative substitutions.

"Conservative (amino acid) substitution" refers to the substitution of amino acid residues with amino acid residues having similar side chains. The family of amino acid residues having similar side chains has been defined in the art, including basic side chains (*e.g.,* lysine, arginine, histidine), acidic side chains (*e.g.,* aspartic acid, glutamic acid), uncharged polar side chains (*e.g.,* glycine, asparagine, glutamine, serine, threonine, tyrosine, cysteine), non-polar side chains (*e.g.,* alanine, valine, leucine, isoleucine, proline, phenylalanine, methionine, tryptophan), β-branched side chains (*e.g.,* threonine, valine, isoleucine), and aromatic side chains (*e.g.,* tyrosine, phenylalanine, tryptophan, histidine). Thus, a non-essential amino acid residue in an immunoglobulin polypeptide is preferably replaced with another amino acid residue from the same side chain family. In another embodiment, a string of amino acids can be replaced with a structurally similar string that differs in order and/or composition of side chain family members.

It will also be understood by those skilled in the art that the antibodies disclosed herein may be modified such that they vary in amino acid sequence from the naturally occurring binding polypeptide from which they are derived. For example, a polypeptide or amino acid sequence derived from a specified protein may be similar, *e.g.,* have a certain percent identity to the starting sequence, *e.g.,* it may be 60%, 70%, 75%, 80%, 85%, 90%, 95%, 98%, 99%, or the range between any two of these values identical to the starting sequence.

In some embodiments, the antibody comprises an amino acid sequence or one or more moieties not normally associated with the antibody. Exemplary modifications are described in more detail herein. For example, the antibodies disclosed herein may comprise a flexible linker sequence, or may be modified to add a functional moiety (*e.g.,* polyethylene glycol (PEG), a drug, a toxin, or a label).

The antibodies, variants, or derivatives thereof of the present disclosure include modified derivatives, *i.e.,* by the covalent attachment of any type of molecule to the antibody, such that the covalent attachment does not prevent the antibody from binding to the epitope. For example, but not by way of limitation, the antibodies may be modified, e.g., by glycosylation, acetylation, PEGylation, phosphorylation, phosphorylation, amidation, derivatization by known protecting/blocking groups, proteolytic cleavage, or ligation with cellular ligands or other proteins. Any of numerous chemical modifications may be performed by known techniques, including, but not limited to, specific chemical cleavage, acetylation, formylation, metabolic synthesis of tunicamycin, *etc.* Additionally, the antibodies may comprise one or more non-classical amino acids.

In some embodiments, the antibodies may be conjugated to therapeutic agents, prodrugs, peptides, proteins, enzymes, viruses, lipids, biological response modifiers, pharmaceutical agents, or PEG.

The antibodies may be conjugated or fused to therapeutic agents, which may include detectable labels (*e.g.,* radioactive labels), immunomodulators, hormones, enzymes, oligonucleotides, photoactive therapeutic or diagnostic agents, cytotoxic agents (which may be drugs or toxins), ultrasound enhancing agents, non-radioactive labels, combinations thereof, and other such agents known in the art.

The antibody can be detectably labeled by coupling it to a chemiluminescent compound. The presence of the chemiluminescent labeling antigen-binding polypeptide is then determined by detecting the presence of luminescence that arises during a chemical reaction. Examples of particularly useful chemiluminescent labeling compounds are luminol, isoluminol, theromatic acridinium ester, imidazole, acridinium salt, and oxalate ester.

The antibody can also be detectably labeled using fluorescence emitting metals such as ¹⁵²Eu or other metals in the lanthanide series. These metals can be attached to the antibody using metal chelating groups such as diethylenetriaminepentaacetic acid (DTPA) or ethylenediaminetetraacetic acid (EDTA). Techniques for conjugating various moieties to antibodies are well known.

### Humanized antibody and chimeric antibody

In any aspect of the present disclosure, the monoclonal antibody is preferably a humanized antibody or a chimeric antibody.

The humanized antibody is an antibody molecule derived from a non-human antibody that binds to a desired antigen having one or more complementarity determining regions (CDRs) from the non-human species and framework regions from a human immunoglobulin molecule. Typically, framework residues in the human framework region will be substituted with corresponding residues from the CDR donor antibody to alter, preferably improve antigen binding. These framework substitutions are identified by methods well known in the art, for example, by modeling of the interactions of the CDR and framework residues to identify framework residues that are important for antigen binding and sequence comparison to identify unusual framework residues at particular positions. Antibodies can be humanized using a variety of techniques known in the art including, for example, CDR-grafting, veneering or resurfacing, and chain shuffling.

Fully human antibodies are particularly desirable for therapeutic treatment of human patients. Human antibodies can be prepared by a variety of methods known in the art, including phage display methods using antibody libraries derived from human immunoglobulin sequences.

Human antibodies can also be produced using transgenic mice which are incapable of expressing functional endogenous immunoglobulins but can express human immunoglobulin genes. For example, human heavy and light chain immunoglobulin gene complexes may be introduced into mouse embryonic stem cells randomly or by homologous recombination. Alternatively, the human variable region, constant region, and diversity region may be introduced into mouse embryonic stem cells in addition to the human heavy and light chain genes. The mouse heavy chain and light chain immunoglobulin genes may be rendered non-functional separately or simultaneously with the introduction of human immunoglobulin loci by homologous recombination. In particular, homozygous deletion of the JH region prevents the production of endogenous antibodies. The modified embryonic stem cells are expanded and microinjected into blastocysts to produce chimeric mice. Chimeric mice are then bred to produce homozygous offspring expressing human antibodies. Transgenic mice are immunized in the normal fashion with a selected antigen, such as all or a portion of a desired target polypeptide. Monoclonal antibodies directed against the antigen can be obtained from immunized transgenic mice using conventional hybridoma technology. Human immunoglobulin transgenes harbored by transgenic mice are rearranged during B cell differentiation, followed by class switching and somatic mutation. Thus, using such a technique, it is possible to produce therapeutically useful IgG, IgA, IgM, and IgE antibodies.

Fully human antibodies which recognize a selected epitope can also be generated using a technique referred to as "guided selection". In this method, a non-human monoclonal antibody, such as a mouse antibody, is selected to guide the selection of a fully human antibody recognizing the same epitope.

DNA encoding desired monoclonal antibodies can be readily isolated and sequenced using conventional procedures (*e.g.,* by using oligonucleotide probes that are capable of specifically binding to genes encoding the heavy and light chains of murine antibodies). The isolated and subcloned hybridoma cells serve as a preferred source of such DNA. Once isolated, the DNA can be placed into an expression vector, which is then transfected into prokaryotic or eukaryotic host cells, such as *E. coli* cells, simian COS cells, Chinese hamster ovary (CHO) cells, or myeloma cells that do not produce immunoglobulins. More specifically, the isolated DNA can be used to clone constant region and variable region sequences for the production of antibodies. Essentially, this entails extraction of RNA from the selected cells, conversion to cDNA, and amplification by PCR using Ig-specific primers. As described herein, transformed cells expressing the desired antibody may be grown in relatively large quantities to provide clinical and commercial supply of immunoglobulins.

Additionally, using conventional recombinant DNA techniques, one or more CDRs of the antigen-binding polypeptide of the present disclosure may be inserted into framework regions, *e.g.,* into human framework regions to humanize a non-human antibody. The framework regions may be naturally occurring or consensus framework regions, and preferably human framework regions. Preferably, the polynucleotide generated by the combination of the framework regions and CDRs encodes an antibody that specifically binds to at least one epitope of a desired polypeptide (*e.g.,* LIGHT). Preferably, one or more amino acid substitutions may be made within the framework regions, and preferably, the amino acid substitutions improve the binding of an antibody to its antigen. In addition, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present disclosure and within the skill of the art.

In addition, techniques developed for the production of "chimeric antibodies" by splicing genes from a mouse antibody molecule with appropriate antigen specificity, as well as genes from a human antibody molecule with appropriate biological activity can be used. As used herein, chimeric antibodies are molecules in which different portions are derived from different animal species, such as molecules having a variable region derived from a murine monoclonal antibody and a human immunoglobulin constant region.

Another efficient method for producing recombinant antibodies is to result in generation of primate antibodies comprising a monkey variable domain and a human constant sequence.

Accordingly, the present disclosure provides a humanized ASGR1 monoclonal antibody; the monoclonal antibody comprises a light chain variable region and a heavy chain variable region, and is selected from any one of the following antibodies:
(a) the light chain variable region comprises LCDR1, LCDR2, and LCDR3 comprising the sequences as shown in SEQ ID NO: 4 to 6, respectively, or an equivalent of each; the heavy chain variable region comprises HCDR1, HCDR2, and HCDR3 comprising the sequences as shown in SEQ ID NO: 7 to 9, respectively, or an equivalent of each;
(b) the light chain variable region comprises LCDR1, LCDR2, and LCDR3 comprising the sequences as shown in SEQ ID NO: 10 to 12, respectively, or an equivalent of each; the heavy chain variable region comprises HCDR1, HCDR2, and HCDR3 comprising the sequences as shown in SEQ ID NO: 13 to 15, respectively, or an equivalent of each;
(c) the light chain variable region comprises LCDR1, LCDR2, and LCDR3 comprising the sequences as shown in SEQ ID NO: 16 to 18, respectively, or an equivalent of each; the heavy chain variable region comprises HCDR1, HCDR2, and HCDR3 comprising the sequences as shown in SEQ ID NO: 19 to 21, respectively, or an equivalent of each; and
(d) the light chain variable region comprises LCDR1, LCDR2, and LCDR3 comprising the sequences as shown in SEQ ID NO: 22 to 24, respectively, or an equivalent of each; the heavy chain variable region comprises HCDR1, HCDR2, and HCDR3 comprising the sequences as shown in SEQ ID NO: 25 to 27, respectively, or an equivalent of each.

In some embodiments, the humanized ASGR1 monoclonal antibody comprises a light chain variable region and a heavy chain variable region; the light chain variable region comprises LCDR1, LCDR2, and LCDR3 comprising the sequences as shown in SEQ ID NO: 4 to 6, respectively, or an equivalent of each; the heavy chain variable region comprises HCDR1, HCDR2, and HCDR3 comprising the sequences as shown in SEQ ID NO: 7 to 9, respectively, or an equivalent of each.

Accordingly, the present disclosure provides a chimeric ASGR1 monoclonal antibody; the monoclonal antibody comprises a light chain variable region and a heavy chain variable region as well as a human constant region, and is selected from any one of the following antibodies:
(e) the light chain variable region comprises the sequence as shown in SEQ ID NO: 28 or an equivalent thereof; the heavy chain variable region comprises the sequence as shown in SEQ ID NO: 29 or an equivalent thereof;
(f) the light chain variable region comprises the sequence as shown in SEQ ID NO: 30 or an equivalent thereof; the heavy chain variable region comprises the sequence as shown in SEQ ID NO: 31 or an equivalent thereof;
(g) the light chain variable region comprises the sequence as shown in SEQ ID NO: 32 or an equivalent thereof; the heavy chain variable region comprises the sequence as shown in SEQ ID NO: 33 or an equivalent thereof; and
(h) the light chain variable region comprises the sequence as shown in SEQ ID NO: 34 or an equivalent thereof; the heavy chain variable region comprises the sequence as shown in SEQ ID NO: 35 or an equivalent thereof.

In some embodiments, the chimeric ASGR1 monoclonal antibody comprises a light chain variable region and a heavy chain variable region; the light chain variable region comprises the sequence as shown in SEQ ID NO: 28 or an equivalent thereof; the heavy chain variable region comprises the sequence as shown in SEQ ID NO: 29 or an equivalent thereof.

Any of the chimeric ASGR1 monoclonal antibodies or humanized ASGR1 monoclonal antibodies described above may comprise a heavy chain constant region such as the human IgG1 heavy chain constant region as shown in SEQ ID NO: 37 and a light chain constant region such as the human Igκ light chain constant region as shown in SEQ ID NO: 38.

### ASGR1 inhibitor

Asialoglycoprotein receptor 1 (ASGR1) is primarily located in liver cells. ASGR1 is a single transmembrane protein comprising a cytoplasmic end, a transmembrane segment, a hinge region, and a carbohydrate-binding region. Glycoprotein in the serum is processed by neuraminidase to produce desialylated glycoprotein, which subsequently binds to ASGR1 on the cell membrane to initiate endocytosis and enters the endosomal pathway. In the acidic environment of the endosome, the receptor is dissociated from the ligand, the proteins are transported to the lysosome for degradation, and ASGR1 is recycled to the cell surface for reuse.

Human ASGR1 consists of 291 amino acids with a molecular weight of 33,186 Da, whose amino acid sequence is shown in SEQ ID NO: 1 (UniProtKB/Swiss-Prot: P07306.2). ASGR1 binds to a ligand in the blood, *i.e.,* asialoglycoprotein, and then enters the lysosome for degradation by means of clathrin-mediated endocytosis. The cytoplasmic end of human ASGR1 is short (1 to 40 aa), the transmembrane region is 40 to 60 aa, and the extracellular domain is further divided into the stalk region (62 to 141 aa) and the carbohydrate-binding region (142 to 291, SEQ ID NO: 2). The gene encoding human ASGR1 is listed in NCBI with gene ID of 432, and the coding region sequence is shown in SEQ ID NO: 36 (NCBI Reference Sequence: NM_001671.5).

As used herein, an "ASGR1 inhibitor" refers to a substance that can reduce or block the binding of ASGR1 to a natural ligand thereof and/or the endocytosis of ASGR1, as well as a substance that can reduce or block the expression of a gene encoding ASGR1.

In some embodiments, the ASGR1 inhibitor described herein may include: (1) an inhibitor that binds to ASGR1; (2) an inhibitor that binds to a ligand of ASGR1 (*e.g.,* asialoglycoprotein); (3) an inhibitor that reduces or blocks the binding of ASGR1 to a ligand thereof; (4) an inhibitor that reduces or blocks the endocytosis of ASGR1; (5) an inhibitor that reduces ASGR1 protein level; (6) an inhibitor that reduces or blocks ASGR1 protein activity; and (7) an inhibitor that reduces or blocks the expression of a gene encoding ASGR1.

In some embodiments, the ASGR1 inhibitor of the present disclosure may be a small molecule compound, a nucleic acid targeting the gene encoding ASGR1, an ASGR1-targeting nucleic acid aptamer, an anti-ASGR1 antibody, or a combination thereof.

In some embodiments, the ASGR1 inhibitor of the present disclosure is an ASGR1 antibody. In some embodiments, the ASGR1 antibody is an ASGR1 monoclonal antibody or antigen-binding fragment thereof.

In some embodiments, the ASGR1 monoclonal antibody binds to human ASGR1 comprising the sequence as shown in SEQ ID NO: 1 and inhibits the binding of human ASGR1 to a natural ligand thereof (*e.g.,* asialoglycoprotein) and/or the endocytosis of human ASGR1.

In some embodiments, the ASGR1 monoclonal antibody binds to the carbohydrate-binding region of ASGR1 and inhibits the binding of human ASGR1 to a natural ligand thereof (e.g., asialoglycoprotein) and/or the endocytosis of human ASGR1. In some embodiments, the carbohydrate-binding region of ASGR1 comprises, or consists essentially of, or consists of the sequence of SEQ ID NO: 2. In some embodiments, the carbohydrate-binding region of ASGR1 comprises, or consists essentially of, or consists of the sequence of SEQ ID NO: 3.

In some embodiments, the ASGR1 monoclonal antibody binds to an epitope comprising one or more of Q240, D242, W244, E253, N265, D266, D267, R237, N209, H257, T259, and Y273 in SEQ ID NO: 1. In some embodiments, the ASGR1 monoclonal antibody binds to an epitope comprising one or more of Q240, D242, W244, E253, N265, and D266 in SEQ ID NO: 1. In some embodiments, the ASGR1 monoclonal antibody binds to an epitope comprising Q240, D242, W244, E253, N265, and D266 in SEQ ID NO: 1.

In some embodiments, the ASGR1 monoclonal antibody is a fully human antibody, a humanized antibody, or a chimeric antibody. In some embodiments, the ASGR1 monoclonal antibody is a fully human antibody or a humanized antibody. In some embodiments, the ASGR1 monoclonal antibody is a chimeric antibody.

In some embodiments, the ASGR1 monoclonal antibody is an antibody described in the above "ASGR1 monoclonal antibody" section; for example, the monoclonal antibody comprises a light chain variable region and a heavy chain variable region, and is selected from any one of the following antibodies:
(a) the light chain variable region comprises LCDR1, LCDR2, and LCDR3 comprising the sequences as shown in SEQ ID NO: 4 to 6, respectively, or an equivalent of each; the heavy chain variable region comprises HCDR1, HCDR2, and HCDR3 comprising the sequences as shown in SEQ ID NO: 7 to 9, respectively, or an equivalent of each;
(b) the light chain variable region comprises LCDR1, LCDR2, and LCDR3 comprising the sequences as shown in SEQ ID NO: 10 to 12, respectively, or an equivalent of each; the heavy chain variable region comprises HCDR1, HCDR2, and HCDR3 comprising the sequences as shown in SEQ ID NO: 13 to 15, respectively, or an equivalent of each;
(c) the light chain variable region comprises LCDR1, LCDR2, and LCDR3 comprising the sequences as shown in SEQ ID NO: 16 to 18, respectively, or an equivalent of each; the heavy chain variable region comprises HCDR1, HCDR2, and HCDR3 comprising the sequences as shown in SEQ ID NO: 19 to 21, respectively, or an equivalent of each;
(d) the light chain variable region comprises LCDR1, LCDR2, and LCDR3 comprising the sequences as shown in SEQ ID NO: 22 to 24, respectively, or an equivalent of each; the heavy chain variable region comprises HCDR1, HCDR2, and HCDR3 comprising the sequences as shown in SEQ ID NO: 25 to 27, respectively, or an equivalent of each;
(e) the light chain variable region comprises the sequence as shown in SEQ ID NO: 28 or an equivalent thereof; the heavy chain variable region comprises the sequence as shown in SEQ ID NO: 29 or an equivalent thereof;
(f) the light chain variable region comprises the sequence as shown in SEQ ID NO: 30 or an equivalent thereof; the heavy chain variable region comprises the sequence as shown in SEQ ID NO: 31 or an equivalent thereof;
(g) the light chain variable region comprises the sequence as shown in SEQ ID NO: 32 or an equivalent thereof; the heavy chain variable region comprises the sequence as shown in SEQ ID NO: 33 or an equivalent thereof; and
(h) the light chain variable region comprises the sequence as shown in SEQ ID NO: 34 or an equivalent thereof; the heavy chain variable region comprises the sequence as shown in SEQ ID NO: 35 or an equivalent thereof.

Other exemplary ASGR1 monoclonal antibodies include those described in WO2017058944A1, WO2022006327A1, or US20210130473A1, the contents of which are incorporated herein by reference in their entirety.

In some embodiments, the ASGR1 inhibitor of the present disclosure is a nucleic acid that targets a DNA or mRNA of ASGR1 and inhibits the expression of ASGR1.

In some embodiments, the nucleic acid is selected from an antisense oligonucleotide (ASO), an siRNA, an shRNA, and a gRNA.

In some embodiments, the nucleic acid is an ASO, which can be modified on a backbone, glycosyl, or base to resist degradation *in vivo.* Suitable modifications include, but are not limited to, phosphorothioate (PSP), phosphorodiamidate morpholino oligomer (PMO), 2'-O-methoxyethyl (2'-MOE), and 5-methylcytosine (5mC).

In some embodiments, the nucleic acid is an siRNA or shRNA, and the siRNA can be delivered by a suitable vector. The suitable vector is, for example, GalNAc, LNP, or AAV.

In some embodiments, the nucleic acid targets the sequence as shown in SEQ ID NO: 36 and inhibits the expression of the gene encoding ASGR1.

In some embodiments, the nucleic acid is a gRNA, which forms a gene editing system with a CRISPR/Cas enzyme (*e.g.,* CRISPR/Cas9) to inhibit or block the expression of the gene encoding ASGR1.

On the premise that the gene encoding ASGR1 is known, those skilled in the art can obtain and screen the sequence of the nucleic acid described above using conventional techniques.

In some embodiments, the nucleic acid (*e.g.,* ASO, siRNA, shRNA, or gRNA) targets the sequence as shown in SEQ ID NO: 36 and inhibits the expression of the gene encoding ASGR1.

In some embodiments, the ASGR1 inhibitor of the present disclosure is a nucleic acid aptamer that targets human ASGR1. In some embodiments, the nucleic acid aptamer binds to human ASGR1 and inhibits the binding of human ASGR1 to a natural ligand thereof (*e.g.,* asialoglycoprotein) and/or the endocytosis of human ASGR1.

### Therapeutic method and use

One aspect of the present disclosure provides a method for reducing the level of cholesterol in the blood, comprising administering to a subject a therapeutically effective amount of any one of the monoclonal antibodies or antigen-binding fragments thereof as described above.

Another aspect of the present disclosure provides a method for reducing the level of triglyceride in the blood, comprising administering to a subject a therapeutically effective amount of any one of the monoclonal antibodies or antigen-binding fragments thereof as described above.

Still another aspect of the present disclosure provides a method for treating and/or preventing a cardiovascular disease such as atherosclerosis, stroke, myocardial infarction, or coronary artery disease, comprising administering to a subject a therapeutically effective amount of any one of the monoclonal antibodies or antigen-binding fragments thereof as described above.

Accordingly, another aspect of the present disclosure provides a use of any one of the monoclonal antibodies or antigen-binding fragments thereof as described above in the manufacture of a medicament for reducing the level of cholesterol in the blood.

Accordingly, another aspect of the present disclosure provides a use of any one of the monoclonal antibodies or antigen-binding fragments thereof as described above in the manufacture of a medicament for reducing the level of triglyceride in the blood.

Accordingly, another aspect of the present disclosure provides a use of any one of the monoclonal antibodies or antigen-binding fragments thereof as described above in the manufacture of a medicament for treating and/or preventing a cardiovascular disease.

Accordingly, another aspect of the present disclosure provides any one of the monoclonal antibodies or antigen-binding fragments thereof as described above for use in reducing the level of cholesterol in the blood.

Accordingly, another aspect of the present disclosure provides any one of the monoclonal antibodies or antigen-binding fragments thereof as described above for use in reducing the level of triglyceride in the blood.

Accordingly, another aspect of the present disclosure provides any one of the monoclonal antibodies or antigen-binding fragments thereof as described above for use in treating and/or preventing a cardiovascular disease.

In another aspect, the inventor has found that co-administration of an ASGR1 inhibitor with a lipid-lowering agent results in a significant synergistic lipid-lowering effect, with a significant reduction in the level of total cholesterol and triglyceride in the serum and liver.

In some embodiments, the present disclosure provides a method for reducing the level of total cholesterol in the blood and/or liver, comprising administering to a subject a therapeutically effective amount of the ASGR1 inhibitor described herein and a therapeutically effective amount of a second lipid-lowering agent.

In some embodiments, the present disclosure provides a method for reducing the level of triglyceride in the blood and/or liver, comprising administering to a subject a therapeutically effective amount of the ASGR1 inhibitor described herein and a therapeutically effective amount of a second lipid-lowering agent.

In some embodiments, the present disclosure provides a method for treating and/or preventing a cardiovascular disease, comprising administering to a subject a therapeutically effective amount of the ASGR1 inhibitor described herein and a therapeutically effective amount of a second lipid-lowering agent.

Accordingly, in some embodiments, the present disclosure provides a use of a combination of the ASGR1 inhibitor and the second lipid-lowering agent described herein in the manufacture of a medicament for reducing the level of total cholesterol in the blood and/or liver.

Accordingly, in some embodiments, the present disclosure provides a use of a combination of the ASGR1 inhibitor and the second lipid-lowering agent described herein in the manufacture of a medicament for reducing the level of triglyceride in the blood and/or liver.

Accordingly, in some embodiments, the present disclosure provides a use of a combination of the ASGR1 inhibitor and the second lipid-lowering agent described herein in the manufacture of a medicament for treating and/or preventing a cardiovascular disease.

Accordingly, in some embodiments, the present disclosure provides a combination of the ASGR1 inhibitor and the second lipid-lowering agent described herein for use in reducing the level of total cholesterol in the blood and/or liver.

Accordingly, in some embodiments, the present disclosure provides a combination of the ASGR1 inhibitor and the second lipid-lowering agent described herein for use in reducing the level of triglyceride in the blood and/or liver.

Accordingly, in some embodiments, the present disclosure provides a combination of the ASGR1 inhibitor and the second lipid-lowering agent described herein for use in treating and/or preventing a cardiovascular disease.

In any one of the preceding embodiments, the second lipid-lowering agent is preferably an HMGCR inhibitor, an ACL inhibitor, a NPC1L1 inhibitor, and/or a PCSK9 inhibitor.

In any one of the preceding embodiments, the second lipid-lowering agent may be administered simultaneously or sequentially with the ASGR1 inhibitor. For example, the second lipid-lowering agent may be administered to a subject prior to administration of the ASGR1 inhibitor, or the second lipid-lowering agent may be administered to a subject after administration of the ASGR1 inhibitor.

In some embodiments, the HMGCR inhibitor is a statin. In some embodiments, the statin is selected from lovastatin, simvastatin, pravastatin, fluvastatin, atorvastatin, rosuvastatin and pitavastatin. In some embodiments, the statin is atorvastatin.

In some embodiments, the ACL inhibitor is bempedoic acid. In some embodiments, the NPC1L1 inhibitor is Ezetimibe.

In some embodiments, the PCSK9 inhibitor is a PCSK9 antibody or a nucleic acid (e.g., siRNA or shRNA) targeting a gene encoding PCSK9. In some embodiments, the PCSK9 antibody is Evolocumab, Alirocumab, or Inclisiran. Other exemplary PCSK9 inhibitors include those described in WO2017220701A1, WO2012088313A1, WO2009026558A1, WO2009102427A2, or WO2017035340A1, the contents of which are incorporated herein by reference in their entirety.

Suitable routes of administration include parenteral administration (e.g., intramuscular, intravenous, or subcutaneous administration) and oral administration. Other conventional modes of administration include intratracheal administration, oral ingestion, inhalation, topical application, or percutaneous, subcutaneous, intraperitoneal, or intraarterial injection.

The appropriate dose is determined by the clinician based on parameters or factors known or suspected to affect treatment or expected to affect treatment in the art. Typically, the dose begins with an amount slightly lower than the optimal dose and is increased in small increments thereafter until the desired or optimal effect is achieved relative to any adverse side effects. Important monitoring indicators include measurements such as inflammatory symptoms or levels of inflammatory cytokines produced.

### Pharmaceutical composition

One aspect of the present disclosure provides a pharmaceutical composition comprising a therapeutically effective amount of any one of the monoclonal antibodies or antigen-binding fragments thereof, and a pharmaceutically acceptable carrier.

Another aspect of the present disclosure provides a pharmaceutical composition comprising a therapeutically effective amount of the ASGR1 inhibitor described herein, a therapeutically effective amount of a second lipid-lowering agent, and a pharmaceutically acceptable carrier.

In any one of the preceding aspects, the pharmaceutical composition is used to reduce the level of total cholesterol in the blood and/or liver. In some embodiments, the pharmaceutical composition is used to reduce the level of triglyceride in the blood and/or liver. In some embodiments, the pharmaceutical composition is used to treat and/or prevent a cardiovascular disease, such as atherosclerosis, stroke, myocardial infarction, or coronary artery disease.

Another aspect of the present disclosure provides a kit comprising a first pharmaceutical composition and a second pharmaceutical composition, wherein the first pharmaceutical composition comprises a therapeutically effective amount of the ASGR1 inhibitor described herein and a pharmaceutically acceptable carrier; the second pharmaceutical composition comprises a therapeutically effective amount of a second lipid-lowering agent and a pharmaceutically acceptable carrier.

In any one of the preceding aspects, the second lipid-lowering agent is preferably an HMGCR inhibitor, an ACL inhibitor, a NPC1L1 inhibitor, and/or a PCSK9 inhibitor. In some embodiments, the HMGCR inhibitor is a statin. In some embodiments, the statin is selected from lovastatin, simvastatin, pravastatin, fluvastatin, atorvastatin, rosuvastatin and pitavastatin. In some embodiments, the statin is atorvastatin. In some embodiments, the ACL inhibitor is bempedoic acid. In some embodiments, the NPC1L1 inhibitor is Ezetimibe. In some embodiments, the PCSK9 inhibitor is an anti-PCSK9 antibody or a nucleic acid (e.g., siRNA or shRNA) targeting a gene encoding PCSK9. In some embodiments, the PCSK9 antibody is Evolocumab, Alirocumab, or Inclisiran.

In order to prepare pharmaceutical or sterile compositions, the drug is mixed with a pharmaceutically acceptable carrier or excipient. Formulations in the form of, *e.g.,* lyophilized powders, slurries, aqueous solutions, or suspensions may be prepared by mixing with physiologically acceptable carriers, excipients, or stabilizers. The pharmaceutically acceptable carrier is well known in the art. It is known in the art how to prepare aqueous compositions comprising active components. Typically, these compositions are prepared as injections or sprays, such as liquid solutions or suspensions; they may also be prepared in solid forms suitable for formulation into solutions or suspensions prior to injection or spraying.

### Sequence Listing

| SEQ ID NO: | Description | Sequence |
|---|---|---|
| 1 | Human ASGR1 | MTKEYQDLQHLDNEESDHHQLRKGPPPPQPLLQRLCS GPRLLLLSLGLSLLLLVVVCVIGSQNSQLQEELRGLRET FSNFTASTEAQVKGLSTQGGNVGRKMKSLESQLEKQQ KDLSEDHSSLLLHVKQFVSDLRSLSCQMAALQGNGSER TCCPVNWVEHERSCYWFSRSGKAWADADNYCRLEDA HLVVVTSWEEQKFVQHHIGPVNTWMGLHDQNGPWK WVDGTDYETGFKNWRPEQPDDWYGHGLGGGEDCAH FTDDGRWNDDVCQRPYRWVCETELDKASQEPPLL |
| 2 | Human ASGR1 carbohydrate recognition domain | AALQGNGSERTCCPVNWVEHERSCYWFSRSGKAWAD ADNYCRLEDAHLVVVTSWEEQKFVQHHIGPVNTWMG LHDQNGPWKWVDGTDYETGFKNWRPEQPDDWYGHG LGGGEDCAHFTDDGRWNDDVCQRPYRWVCETELDKA SQEPPLL |
| 3 | Human ASGR1 carbohydrate recognition domain epitope | RLEDAHLVVVTSWEEQKFVQHHIGPVNTWMGLHDQN GPWKWVDGTDYETGFKNWRPEQPDDWYGHGLGGGE DCAHFTDDGRWNDDVCQRPYRW |
| 4 | 4B9-LCDR1 | QSVYNNKN |
| 5 | 4B9-LCDR2 | YAS |
| 6 | 4B9-LCDR3 | QGEFSCSSADCCA |
| 7 | 4B9-HCDR1 | GFSFSGSLW |
| 8 | 4B9-HCDR2 | IYVGSSGST |
| 9 | 4B9-HCDR3 | ARRYTDAYGGYPAGSFVFKL |
| 10 | 3E8-LCDR1 | QKISNE |
| 11 | 3E8-LCDR2 | RAS |
| 12 | 3E8-LCDR3 | QCTYGSSNIVNYGGA |
| 13 | 3E8-HCDR1 | GFSLSSNA |
| 14 | 3E8-HCDR2 | IVSSGAA |
| 15 | 3E8-HCDR3 | VRGNL |
| 16 | 3E12-LCDR1 | ESISSY |
| 17 | 3E12-LCDR2 | QAS |
| 18 | 3E12-LCDR3 | QSNYGTTSSTYDT |
| 19 | 3E12-HCDR1 | GFSFNKKYY |
| 20 | 3E12-HCDR2 | IYAGSSGST |
| 21 | 3E12-HCDR3 | ARGYYYYNGYVYYGYGMDL |
| 22 | 5E5-LCDR1 | ESISSY |
| 23 | 5E5-LCDR2 | DAS |
| 24 | 5E5-LCDR3 | QQGYSGNNLDNA |
| 25 | 5E5-HCDR1 | GFSLSSYA |
| 26 | 5E5-HCDR2 | IYASGST |
| 27 | 5E5-HCDR3 | ARGDDSYTTDDL |
| 28 | 4B9-VL | QVLTQTPSPVSAAVGGTVTINCQASQSVYNNKNLGWY QQKPGQPPKLLIYYASDLASGVPSRFKGSGSGKQFTLTI SDVQCDDAATYYCQGEFSCSSADCCAFGGGTEVVVK |
| 29 | 4B9-VH | QSLEESGGDLVKPGATMTLTCKASGFSFSGSLWICWVR QAPGKGLEWIACIYVGSSGSTYYASWAKGRFTISTTSST TVTLQMTSLTAADTATYFCARRYTDAYGGYPAGSFVFK LWGPGTLVTVSS |
| 30 | 3E8-VL | DVVMTQTPASVEAAVGGTVTIKCQASQKISNELSWYQ QKPGQPPKRLIYRASTLESGVPSRFKGSGSGTEFTLTISD LECADAATYYCQCTYGSSNIVNYGGAFGGGTEVVVK |
| 31 | 3E8-VH | QSVEESGGRLVTPGTPLTLTCTVSGFSLSSNAINWVRQA PGKGLEWIGYIVSSGAAYYASWAKGRFTISKTSTTVDLS ITSPTTEDTATYFCVRGNLWGPGTLVTVSS |
| 32 | 3E12-VL | DIVMTQTPASVEAAVGGTITINCQASESISSYLSWYQQK PGQRPKLLIYQASKLESGVPSRFKGSGSGTEYTLTISDLE CADAATYYCQSNYGTTSSTYDTFGGGTEVVVK |
| 33 | 3E12-VH | QEQLEESGGDLVKPEGSLTLTCTASGFSFNKKYYMCWV RQAPGKGLEWIACIYAGSSGSTYYASWVNGRFTISQTSS TTVTLQMTSLTAADTATYFCARGYYYYNGYVVYYGYG MDLWGPGTLVTVSS |
| 34 | 5E5-VL | AYDMTQTPASVEVAVGGTVTIKCQASESISSYLNWYQQ KPGQRPKVLIYDASDLASGVSSRFKGSGSGTQFTLTISG VQCDDAATYYCQQGYSGNNLDNAFGGGTEVVVK |
| 35 | 5E5-VH | QSVEESGGRLVTPGTPLTLTCTVSGFSLSSYAMSWVRQA PGKGLEYIGIIYASGSTYYASWAKGRFTISKTSTTVDLK MTSPTTEDTATYFCARGDDSYTTDDLWGPGTLVTVSS |
| 36 | ASGR1-mRNA | ACACAGACACGCAGACACAGAGACACCGGGGCCCA GGGCCCTCCTATGGACCCTGCCCGCTCCCCTCCCATT GTCCACGGCTGTCCGCCCACCCCCATTCTCCAAGCTT CAGCCCCCTCCTTAGTTCGGCATCTGCACAGCACTGA AGAACCTGGGAATCAGACCCTGAGACCCTGAGCAAT CCCAGGTCCAGCGCCAGCCCTATCATGACCAAGGAGT ATCAAGACCTTCAGCATCTGGACAATGAGGAGAGTG ACCACCATCAGCTCAGAAAAGGGCCACCTCCTCCCC AGCCCCTCCTGCAGCGTCTCTGCTCCGGACCTCGCCT |
| | | CCTCCTGCTCTCCCTGGGCCTCAGCCTCCTGCTGCTT GTGGTTGTCTGTGTGATCGGATCCCAAAACTCCCAGC TGCAGGAGGAGCTGCGGGGCCTGAGAGAGACGTTCA GCAACTTCACAGCGAGCACGGAGGCCCAGGTCAAGG GCTTGAGCACCCAGGGAGGCAATGTGGGAAGAAAGA TGAAGTCGCTAGAGTCCCAGCTGGAGAAACAGCAGA AGGACCTGAGTGAAGATCACTCCAGCCTGCTGCTCC ACGTGAAGCAGTTCGTGTCTGACCTGCGGAGCCTGA GCTGTCAGATGGCGGCGCTCCAGGGCAATGGCTCAG AAAGGACCTGCTGCCCGGTCAACTGGGTGGAGCACG AGCGCAGCTGCTACTGGTTCTCTCGCTCCGGGAAGGC CTGGGCTGACGCCGACAACTACTGCCGGCTGGAGGA CGCGCACCTGGTGGTGGTCACGTCCTGGGAGGAGCA GAAATTTGTCCAGCACCACATAGGCCCTGTGAACACC TGGATGGGCCTCCACGACCAAAACGGGCCCTGGAAG TGGGTGGACGGGACGGACTACGAGACGGGCTTCAAG AACTGGAGGCCGGAGCAGCCGGACGACTGGTACGGC CACGGGCTCGGAGGAGGCGAGGACTGTGCCCACTTC ACCGACGACGGCCGCTGGAACGACGACGTCTGCCAG AGGCCCTACCGCTGGGTCTGCGAGACAGAGCTGGAC AAGGCCAGCCAGGAGCCACCTCTCCTTTAATTTATTT CTTCAATGCCTCGACCTGCCGCAGGGGTCCGGGATTG GGAATCCGCCCATCTGGGGGCCTCTTCTGCTTTCTCG GGAATTTTCATCTAGGATTTTAAGGGAAGGGGAAGGA TAGGGTGATGTTCCGAAGGTGAGGAGCTTGAAACCC GTGGCGCTTTCTGCAGTTTGCAGGTTATCATTGTGAA CTTTTTTTTTTTAAGAGTAAAAAGAAATATACCTAAA |
| 37 | Human IgG1 heavy chain constant region (Uniprot: P01857) | |
| 38 | Human Igκ light chain constant region (Uniprot: P01834) | |

### Examples

### Materials and methods

### Mice

CRISPR/Cas9-mediated *Asgr1* systemic knock-out mice were constructed by Chengdu GemPharmatech Co., Ltd, in which cleavage between introns 2 and 3 as well as introns 8 and 9 was performed by sgRNA-mediated Cas9 endonuclease. A partial deletion between exons 3 and 8 was achieved through homologous recombination, resulting in *Asgr1* systemic knockout mice.

In the experiment, wild-type littermates were used as a control. All mice were housed in an SPF (specific pathogen-free) facility and kept on a 12-hour light/12-hour dark cycle. 8-week-old male or female mice were fed a high-fat, high-cholesterol, and cholate diet (Research Diets, D12109C) as required by the experimental protocols. For treatments involving antibody neutralization or the combination of antibodies with statins or Ezetimibe, the mice were intraperitoneally injected with antibodies or intragastrically administered with drugs at specified doses. All animals were fasted for 4 hours before being sacrificed. All animal experiments were following protocols approved by the Institutional Animal Care and Use Committee of Wuhan University and adhered to the Chinese National Laboratory Animal-Guideline for Ethical Review of Animal Welfare.

### Materials and plasmids

Lovastatin (purity ≥ 98%, HPLC) was purchased from Shanghai Pharm Vally. Sodium mevalonate (#4667), anti-FLAG M2 beads (#A2220), anti-MYC beads (E6654), Fetuin A (SRP6217), D-Galactose (G5388), N-acetyl-D-galactosamine (A-2795), Phenylmethanesulfonyl fluoride (PMSF, #P7626), Protease inhibitor cocktail (#P8340), and β-mercaptoethanol (#M3148) were all purchased from Sigma. Dil (1,1-dioctadecyl-3,3,3,3-tetramethyl-indocarbocyanine perchlorate)-LDL (#20614ES76) was purchased from Shanghai YEASEN. Lipofectamine RNAiMAX (#13778150) was purchased from Thermo Fisher. MG132 (#I-130) was purchased from Boston Biochem. Puromycin (#BS111) was purchased from Biosharp. G418 (#345810), Pepstatin A (#516481), and ALLN (N-acetyl-Leu-Leu-norleucinal, #208719) were purchased from Calbiochem. Ni-NTA Agarose (#30230) was purchased from Qiagen. LPEI (Linear polyethylenimine, #23966-1) was purchased from Polysciences. FuGENE HD (#E2311) and M-MLV RTase (#M1701) were purchased from Promega. Leupeptin (#11034626001) was purchased from Roche. DTT (DL-Dithiothreitol, #A100281) and NP-40 (A100109) were purchased from Shanghai Sangon. Phosphatase inhibitor (P1082) was purchased from Beyotime. Medium supplemented with fetal bovine serum (FBS) for cell culture was purchased from Life Technology. Taq polymerase was purchased from TIANGEN. KOD Hot Start DNA polymerase (#KOD-401; TOYOBO) was purchased from Takara; RNA duplex was synthesized by Guangzhou RiboBio; Q-PCR 2×MIX was purchased from Mona. Total cholesterol (TC), total triglyceride (TG), and bile acid kits were purchased from Nanjing Kehua Bio-engineering. NEFA kit (294-63601) and Phospholipid kit (292-63901) were purchased from WAKO. ALT, AST, and AKP kits were purchased from Nanjing Jiancheng Bioengineering. Blood glucose test strips and meters were purchased from OneTouch. Lipoprotein-deficient serum (d > 1.215 g/mL), *i.e.,* LPPS, was prepared by ultracentrifugation from newborn calf serum.

The following plasmids were constructed using standard molecular cloning techniques: Human and mouse Asgr1, Asgr2, Lxrα, and Lxrβ gene fragments were derived from cDNA reverse-transcribed from RNAin Huh7 cells and mouse liver tissues, and human BARD1 gene fragment was amplified from Huh7 cells. These fragments were cloned into p3×Flag-CMV14, pEGFP-C1, and pcDNA3-C-5×Myc vectors, respectively. pDEST-FRT/T0-GFP-BRCA1 (#71116) was purchased from Addgene. Various truncations and point mutations of ASGR1 were constructed using site-directed mutagenesis.

Huh7 and HEK293T cells were grown as monolayers at 37°C with 5% CO₂. Cells were maintained in Medium A (DMEM containing 100 units/mL penicillin and 100 mg/mL streptomycin) supplemented with 10% fetal bovine serum (FBS). Cholesterol-deficient Medium B was prepared by supplementing Medium A with 5% lipoprotein-depleted serum (LPPS), 1 µM lovastatin, and 10 µM mevalonate. Primary mouse hepatocytes were cultured in Medium D (M199) supplemented with 5% FBS, 100 units/mL penicillin, and 100 mg/mL streptomycin.

### Western blotting

The collected cells or tissues were lysed in RIPA lysis buffer supplemented with protease and phosphatase inhibitors. The RIPA lysis buffer contained 50 mM Tris-HCl (pH = 8.0), 150 mM NaCl, 2 mM MgCl₂, 1.5% NP-40, 0.1% SDS, and 0.5% sodium deoxycholate. The protease inhibitor contained 10 µM MG-132, 10 µg/mL leupeptin, 1 mM PMSF, 5 µg/mL pepstatin, 25 µg/mL ALLN, and 1 mM DTT. The protein concentration of the lysate was determined using a BCA method (Thermo Fisher Scientific). The protein samples were incubated with membrane solubilization buffer (62.5 mM Tris-HCl (pH = 6.8), 15% SDS, 8 M urea, 10% glycerol, and 100 mM DTT) and 4× loading buffer (150 mM Tris-HCl (pH = 6.8), 12% SDS, 30% glycerol, 6% β-mercaptoethanol, and 0.02% bromophenol blue) at 37°C for 30 minutes. The protein samples were separated by SDS-PAGE and transferred to a PVDF membrane, which was blocked in TBST, *i.e.,* TBS containing 0.075% Tween 20 and 5% skim milk (3% BSA for phosphorylation experiments) for 1 hour. The membrane was incubated overnight at 4°C with a specified primary antibody, and washed three times with TBST. Finally, Pierce ECL Plus Western blotting substrate (Thermo Fisher Scientific) was used for detection.

### Primary antibodies used in experiments

Anti-β-actin (#A5441) and anti-FLAG (#F3165) were purchased from Sigma. Anti-AMPK (10929-2-AP), anti-ACC (67373-I-Ig), anti-FASN (10624-2-AP), anti-GAPDH (60004-1-Ig), and anti-ASGR1 (11739-1-AP) were purchased from ProteinTech. Anti-CYP7A1 (sc-518007), anti-SREBP1 (sc-13551), and anti-BRCA1 (sc-6954) were purchased from Santa Cruz. Anti-c-Myc and anti-HMGCR were purified from hybridoma cell lines (ATCC) 9E10 and A9, respectively. Anti-LDLR and anti-H2 (HMGCR) were affinity purified antibodies from antisera which was obtained by immunizing rabbits with soluble segments. Anti-EGFP was obtained by immunizing rabbits with purified EGFP protein expressed in *E. coli.* Anti-LXRα (ab41902) was purchased from Abcam. Anti-LXRβ (NB100-74457), anti-ABCG8 (NBP1-71706F), and anti-ABCA1 (NB400-105) were purchased from Novus. Anti-BARD1 (A300-263A) was purchased from Bethy. Anti-phos ACC (118187) and anti-phos-AMPK (#2535) were purchased from Cell Signaling Tech. Secondary antibodies were purchased from Jackson ImmunoResearch Laboratories.

### Chemical analysis of blood and liver

Blood was collected from the orbital sinus to obtain serum for the measurement of total cholesterol and triglyceride. Liver tissues were homogenized and the supernatant was collected for lipid extraction to obtain total cholesterol in the liver, and triglyceride (TG) and total cholesterol (TC) levels in the liver were measured using an assay kit (Kehua, China). Phospholipid levels were measured using a kit (Phospholipid, WAKO, Japan). ALT and AST levels in the serum were measured using a kit from Nanjing Jiancheng Bioengineering (China).

### Results and analysis

### Example 1: ASGR1 neutralizing antibody 4B9 promoted cholesterol efflux into bile and feces, showing a lipid-lowering therapeutic effect.

ASGR1 can bind to a large number of desialylated glycoproteins. To better simulate the improvement of *Asgr1* knockout on metabolic syndrome induced by high-fat, high-cholesterol conditions, we purified ASGR1 protein from HEK293T cells, which was used to immunize rabbits to obtain a neutralizing antibody blocking ASGR1 function. The specific procedure is shown in FIG. 1a: Steps for preparing ASGR1 monoclonal neutralizing antibody. Purified ASGR1 protein was used as the antigen to immunize rabbits, and after the first round of screening, four B-cell monoclonal antibodies were selected as candidates. The coding region of the variable region of the antibody was then sequenced and cloned into an antibody expression vector, which was transfected into mammalian cells. The efficiency of transfection was confirmed by Western blotting and real-time quantitative PCR. Finally, the rabbit Fc fragment was replaced with a mouse Fc fragment. The most effective neutralizing antibody 4B9 was selected for large-scale production and used in subsequent experiments. The experimental results demonstrated that 4B9 exhibited a potential lipid-lowering effect. 4B9 and the other three ASGR1 monoclonal neutralizing antibodies were able to significantly increase the protein stability of LXRα (FIGs. 1c, 1d), and cholesterol efflux-related genes were also significantly up-regulated (FIG. 1e). Further analysis involved transfecting plasmids with different ASGR1 truncations to determine the segment recognized by 4B9. It was found that 4B9 primarily recognizes the sequence between 182 to 274 aa (FIG. 1f).

We further investigated whether 4B9 was able to completely simulate the improvement of *Asgr1* deletion on high-fat, high-cholesterol conditions. 8-week-old *Asgr1* knockout mice and wild-type littermates were randomly divided into 4 groups as shown in FIG. 2 according to the genotype, with 6 mice per group. The mice were given free access to water and fed a high-fat, high-cholesterol, and cholate diet (60% fat, 1.25% cholesterol, and 0.5% cholate, HF/HC/BS). Meanwhile, the mice were intraperitoneally injected every other day with control antibody or ASGR1 neutralizing antibody 4B9 at a dose of 10 mg/kg/day. After 14 days, the mice were fasted for 4 hours before being sacrificed. As a result, wild-type mice injected with 4B9 antibody showed results consistent with *Asgr1* deletion, i.e., cholesterol efflux-related proteins, such as LXR, ABCG8, ABCA1, and CYP7A1, were significantly increased; p-ACC was also significantly up-regulated; lipid synthesis-related proteins SREBP1 and FASN were significantly reduced; LDLR remained unchanged; BARD1 was significantly reduced; and all parameters remained unchanged in *Asgr1* knockout mice with or without injection of 4B9, indicating that 4B9 specifically targets ASGR1 (FIG. 2a). Real-time quantitative PCR results were also consistent with protein-level changes (FIG. 2b). For biochemical parameters, mice injected with 4B9 antibody showed a significant decrease in total cholesterol and triglyceride in serum (FIGs. 2c, d), a significant decrease in total cholesterol and triglyceride in liver (FIGs. 2e, f), a significant increase in the volume of gallbladder (FIGs. 2g, j), a significant increase in the concentration and total amount of total cholesterol in bile (FIGs. 2h, i), a significant increase in total cholesterol in feces (FIG. 2k), and a significant increase in the concentration and total amount of total bile acid in bile (FIGs. 2l, m), as compared to the control group. There were no significant changes in body weight, daily food intake, ratio of liver weight to body weight, blood glucose, ALT, and AST (FIGs. 2n-s). These results demonstrate that ASGR1 neutralizing antibody 4B9 shows a very significant lipid-lowering effect and can greatly alleviate metabolic syndrome induced by a high-fat, high-cholesterol diet.

### Example 2: The combination of antibody 4B9 and atorvastatin showed a synergistic lipid-lowering effect.

4B9 was shown to significantly alleviate the metabolic syndrome phenotype induced by a high-fat, high-cholesterol diet in previous experiments (FIG. 2). We further investigated whether the combination of 4B9 and atorvastatin could produce a synergistic lipid-lowering effect. 8-week-old *Asgr1* knockout mice and wild-type littermates were randomly divided into 8 groups as shown in FIG. 3 according to the genotype, with 6 mice per group. The mice were given free access to water and fed a high-fat, high-cholesterol, and cholate diet (60% fat, 1.25% cholesterol, and 0.5% cholate, HF/HC/BS).

Meanwhile, the mice were intraperitoneally injected every other day with control antibody or ASGR1 neutralizing antibody 4B9 at a dose of 10 mg/kg/day and were intragastrically administered each day with atorvastatin at a dose of 30 mg/kg/day. After 14 days, the mice were fasted for 4 hours before being sacrificed. The experimental results showed that 4B9 alone still exhibited increased levels of cholesterol efflux-related genes and their proteins, but inhibited the levels of lipid synthesis-related genes and their proteins, without affecting the levels of cholesterol synthesis or absorption-related genes and their proteins, while the combination of atorvastatin and 4B9 did not affect the effect of 4B9 in promoting the expression of cholesterol efflux-related genes and their proteins such as LXRs, ABCG8, ABCA1, and CYP7A1, but was able to more significantly inhibit the expression of lipid synthesis-related genes and their proteins, such as SREBP1 and FASN (FIGs. 3a, 3q-r). 4B9 or atorvastatin alone was able to effectively reduce the levels of total cholesterol and triglyceride in serum and liver, while the combination of 4B9 and atorvastatin exhibited a more significant lipid-lowering effect (FIGs. 3b-e, 3s-t). 4B9 alone increased the total amounts of total cholesterol and total bile acid in bile, while atorvastatin had almost no effect on total cholesterol and total bile acid in bile (FIGs. 3g, i). 4B9 alone significantly increased total cholesterol efflux in feces, while atorvastatin had no effect on cholesterol in feces (FIG. 3j). No significant differences were observed among the 8 groups of mice in terms of body weight, daily food intake, ratio of liver weight to body weight, blood glucose, ALT, and AST, whether 4B9 or atorvastatin was used alone or in combination (FIGs. 3k-p). In summary, the combination of 4B9 and atorvastatin can achieve a better lipid-lowering effect compared to 4B9 or atorvastatin alone.

### Example 3: The combination of antibody 4B9 and Ezetimibe showed a synergistic lipid-lowering effect.

4B9 has been shown to have a certain lipid-lowering effect in mice (FIG. 2), so the combination of 4B9 and EZ was hypothesized to increase the lipid-lowering effect to a greater extent. We then devised an experiment as in FIG. 4 to verify the hypothesis. Mice were randomly divided into 8 groups according to the genotype (n = 6) and fed an HF/HC/BS diet. They were intragastrically administered with EZ at 10 mg/kg per day and were intraperitoneally injected with 4B9 at 10 mg/kg every other day. The mice were sacrificed on day 7 to collect their tissues. The results showed that 4B9 alone still exhibited increased levels of cholesterol efflux-related genes and their proteins such as LXRs, ABCG8, ABCA1, and CYP7A1, but inhibited the levels of lipid synthesis-related genes and their proteins such as SREBP1 and FASN, without affecting the levels of cholesterol synthesis or absorption-related genes and their proteins, while Ezetimibe alone or in combination with 4B9 showed a significant decrease in cholesterol efflux-related genes and their proteins as well as lipid synthesis-related genes and their proteins (FIGs. 4a, 4p-q). 4B9 or Ezetimibe alone was able to effectively reduce the levels of total cholesterol and triglyceride in serum and liver, while the combination of 4B9 and Ezetimibe exhibited a more significant lipid-lowering effect (FIGs. 4b-e, 4r-s). 4B9 alone increased the total amounts of cholesterol and total bile acid in bile, while Ezetimibe alone or in combination with 4B9 significantly inhibited the total amounts of total cholesterol and total bile acid in bile (FIGs. 4g, i). No significant differences were observed among the 8 groups of mice in terms of body weight, daily food intake, ratio of liver weight to body weight, blood glucose, ALT, and AST, whether 4B9 or Ezetimibe was used alone or in combination (FIGs. 4j-o). In summary, the combination of 4B9 and Ezetimibe can achieve a better lipid-lowering effect compared to 4B9 or Ezetimibe alone.

It should be understood that although the present disclosure has been specifically disclosed by preferred embodiments and optional features, those skilled in the art may make modifications, improvements, and variations to the present disclosure disclosed herein, which are considered to be within the scope of the present disclosure. The materials, methods, and examples provided herein are representative and exemplary of preferred embodiments and are not intended to be a limitation on the scope of the present disclosure.

## Claims

1. A monoclonal antibody or an antigen-binding fragment thereof that binds to ASGR1, wherein the monoclonal antibody inhibits or blocks the binding of ASGR1 to a natural ligand thereof and/or the endocytosis of ASGR1; the monoclonal antibody comprises a light chain variable region and a heavy chain variable region, and is selected from any one of the following antibodies:
(a) the light chain variable region comprises LCDR1, LCDR2, and LCDR3 comprising the sequences as shown in SEQ ID NO: 4 to 6, respectively; the heavy chain variable region comprises HCDR1, HCDR2, and HCDR3 comprising the sequences as shown in SEQ ID NO: 7 to 9, respectively;
(b) the light chain variable region comprises LCDR1, LCDR2, and LCDR3 comprising the sequences as shown in SEQ ID NO: 10 to 12, respectively; the heavy chain variable region comprises HCDR1, HCDR2, and HCDR3 comprising the sequences as shown in SEQ ID NO: 13 to 15, respectively;
(c) the light chain variable region comprises LCDR1, LCDR2, and LCDR3 comprising the sequences as shown in SEQ ID NO: 16 to 18, respectively; the heavy chain variable region comprises HCDR1, HCDR2, and HCDR3 comprising the sequences as shown in SEQ ID NO: 19 to 21, respectively;
(d) the light chain variable region comprises LCDR1, LCDR2, and LCDR3 comprising the sequences as shown in SEQ ID NO: 22 to 24, respectively; the heavy chain variable region comprises HCDR1, HCDR2, and HCDR3 comprising the sequences as shown in SEQ ID NO: 25 to 27, respectively;
(e) the light chain variable region comprises the sequence as shown in SEQ ID NO: 28; the heavy chain variable region comprises the sequence as shown in SEQ ID NO: 29;
(f) the light chain variable region comprises the sequence as shown in SEQ ID NO: 30; the heavy chain variable region comprises the sequence as shown in SEQ ID NO: 31;
(g) the light chain variable region comprises the sequence as shown in SEQ ID NO: 32; the heavy chain variable region comprises the sequence as shown in SEQ ID NO: 33; and
(h) the light chain variable region comprises the sequence as shown in SEQ ID NO: 34; the heavy chain variable region comprises the sequence as shown in SEQ ID NO: 35.

2. The monoclonal antibody or the antigen-binding fragment thereof according to claim 1, wherein the monoclonal antibody is a humanized antibody or a chimeric antibody.

3. A pharmaceutical composition comprising a therapeutically effective amount of an ASGR1 inhibitor, a therapeutically effective amount of a second lipid-lowering agent, and a pharmaceutically acceptable carrier.

4. A kit comprising a first pharmaceutical composition and a second pharmaceutical composition, wherein the first pharmaceutical composition comprises a therapeutically effective amount of an ASGR1 inhibitor and a pharmaceutically acceptable carrier; the second pharmaceutical composition comprises a therapeutically effective amount of a second lipid-lowering agent and a pharmaceutically acceptable carrier.

5. A use of a combination of an ASGR1 inhibitor and a second lipid-lowering agent in the manufacture of a medicament for reducing the level of cholesterol and/or triglyceride in the blood or for treating and/or preventing a cardiovascular disease.

6. The pharmaceutical composition, kit, or use according to any one of claims 3 to 5, wherein the ASGR1 inhibitor is selected from an anti-ASGR1 monoclonal antibody or an antigen-binding fragment thereof, a nucleic acid targeting the coding nucleic acid of ASGR1, a nucleic acid aptamer targeting ASGR1, and a combination thereof.

7. The pharmaceutical composition, kit, or use according to claim 6, wherein the anti-ASGR1 monoclonal antibody binds to the sequence as shown in SEQ ID NO: 1, SEQ ID NO: 2, or SEQ ID NO: 3, and inhibits or blocks the binding of ASGR1 to a natural ligand thereof and/or the endocytosis of ASGR1.

8. The pharmaceutical composition, kit, or use according to claim 6, wherein the anti-ASGR1 monoclonal antibody or the antigen-binding fragment thereof is the monoclonal antibody or the antigen-binding fragment thereof according to claim 1.

9. The pharmaceutical composition, kit, or use according to any one of claims 3 to 5, wherein the second lipid-lowering agent is an HMGCR inhibitor, an ATP-citrate lyase inhibitor, a NPC1L1 inhibitor, or a PCSK9 inhibitor.

10. The pharmaceutical composition, kit, or use according to claim 9, wherein the HMGCR inhibitor is a statin.

11. The pharmaceutical composition, kit, or use according to claim 10, wherein the statin is atorvastatin.

12. The pharmaceutical composition, kit, or use according to claim 9, wherein the ATP-citrate lyase inhibitor is bempedoic acid.

13. The pharmaceutical composition, kit, or use according to claim 9, wherein the NPC1L1 inhibitor is Ezetimibe.

14. The pharmaceutical composition, kit, or use according to claim 9, wherein the PCSK9 inhibitor is Evolocumab, Alirocumab, or Inclisiran.

15. The use according to claim 5, wherein the cardiovascular disease is selected from atherosclerosis, stroke, myocardial infarction, and coronary artery disease.
